# EUROPÄISCHE PATENTSCHRIFT

(11) **EP 2 164 963 B1**
(45) Veröffentlichungstag und Bekanntmachung des Hinweises auf die Patenterteilung: **07.05.2014**
(21) Anmeldenummer: 08760239.7
(22) Anmeldetag: 29.05.2008
(51) Int. Cl.: C12N 15/10, C12Q 1/68

(54) **VERFAHREN ZUR STABILISIERUNG EINER BIOLOGISCHEN PROBE**
METHOD FOR STABILISING A BIOLOGICAL SAMPLE
PROCÉDÉ DE STABILISATION D'UN ÉCHANTILLON BIOLOGIQUE

(30) Priorität: 31.05.2007 DE 102007025277
(43) Veröffentlichungstag der Anmeldung: 24.03.2010
(60) Teilanmeldung: 12164227.6
(73) Patentinhaber: Qiagen GmbH, 40724 Hilden (DE)
(72) Erfinder: HOLLÄNDER, Vera, 59423 Unna (DE)
(74) Vertreter: Roth, Carla
(86) Internationale Anmeldenummer: PCT/EP2008/056648
(87) Internationale Veröffentlichungsnummer: WO 2008/145710

(56) Entgegenhaltungen:
- EP-A- 0 578 885
- WO-A-2004/072228

## Beschreibung

### Gebiet der Erfindung

Die vorliegende Erfindung betrifft ein Verfahren zur Stabilisierung einer biologischen Probe, insbesondere zur Stabilisierung von Nukleinsäuren, Proteinen und der Morphologie von biologischen Proben.

### Technischer Hintergrund

Es ist seit langem bekannt, dass die genetische Herkunft und der physiologische Status einer Zelle durch Studien ihres Genoms, ihres Transkriptoms, ihres Proteoms und ihres Methyloms bestimmt und untersucht werden kann.

Der Begriff "Genom" bezeichnet die Gesamtheit der vererbbaren Nukleinsäuren in einer Probe, d.h. einem Lebewesen, einem Gewebe, einer Zelle oder einem Zellkompartiment bzw. einer Biopsie, einem Abstrich, einem Schnitt oder dergleichen. Zumeist handelt es sich bei der vererbbaren Nukleinsäure um DNA.

Der Begriff "Transkriptom" bezeichnet die Summe der zu einem bestimmten Zeitpunkt in einer solchen Probe transkribierten, das heißt von der DNA in RNA überschriebenen, Gene, also die Gesamtheit aller RNA-Moleküle.

Der Begriff "Proteom" bezeichnet hingegen die Gesamtheit aller Proteine in einer solchen Probe.

Der Begriff "Methylom" beschreibt das Methylierungsmuster des Genoms. Es umfasst die Gesamtheit und das Muster der Positionen methylierten Cytosins (mC) einer gesunden DNA.

Die Analyse des Genoms, Transkriptoms, Proteoms und/oder Methyloms ist in vielerlei Belangen indirekten, konventionellen Methoden, wie z. B. dem Nachweis von Stoffwechselprodukten, insbesondere für diagnostische Zwecke potentiell überlegen.

Die Biologie hat in den letzen zwanzig Jahren hierfür ein umfassendes molekularbiologisches Instrumentarium entwickelt. Daher ist für die Zukunft mit einer noch stärkeren Verbreitung molekularbiologischer Analysen zu rechnen, z. B. in der medizinischen und klinischen Diagnostik, in der Forensik, in der Pharmazie bei der Entwicklung und Evaluierung von Arzneimitteln, in der Lebensmittelanalytik sowie bei der Überwachung der Lebensmittelherstellung, in der Agrarwirtschaft bei der Züchtung von Nutzpflanzen und Nutztieren sowie in der Umweltanalytik und in vielen Forschungsgebieten.

Durch die Analyse des Transkriptoms, speziell der mRNA in Zellen, lassen sich die Aktivitäten von Genen direkt bestimmen. Die quantitative Analyse von Transkriptmustem (mRNA-Mustem) in Zellen durch moderne molekularbiologische Methoden, wie z. B. Echtzeit-Reverse-Transkriptase-PCR ("Real time RT PCR") oder Genexpressions-Chip-Analysen ermöglicht z. B. die Erkennung fehlerhaft exprimierter Gene, wodurch z. B. Stoffwechselkrankheiten, Infektionen oder eine etwaige Prädisposition für eine Krebserkrankung erkannt werden können.

Die Analyse des Genoms durch molekularbiologische Methoden, wie z. B. PCR, RFLP, AFLP oder Sequenzierung ermöglicht z. B. den Nachweis genetischer Defekte oder die Bestimmung des HLA-Typs sowie anderer genetischer Marker. Die Analyse genomischer DNA und RNA wird auch zum direkten Nachweis von infektiösen Erregern, wie Viren, Bakterien usw. eingesetzt. Die Analyse des Methyloms erlaubt Aussagen über die Aktivität bestimmter Gene; bestimmte Methylierungsmuster lassen dabei z.B. auf die Prädisposition bezüglich bestimmter Krankheiten schließen.

Dabei ist insbesondere die Kombination von molekularbiologischen und morphologischen Verfahren sehr vielversprechend. So kann z.B. eine Gewebeprobe, bei der morphologisch ein bestimmter Tumortypus diagnostiziert wird, durch Charakterisierung des Genoms, Transkriptoms, Proteoms und/oder Methyloms genauer untersucht werden, um einen Tumor-Subtypus zu bestimmen, was wiederum die Einstellung einer zielgerichteten Therapie ermöglicht.

Unbedingte Voraussetzung für die genannten Untersuchungen ist eine sofortige Stabilisierung der biologischen Probe nach Entnahme aus ihrer natürlichen Umgebung, d.h. die Erhaltung ihrer bei Probennahme bestehenden genomischen, transkriptomischen, methylomischen, proteomischen sowie morphologischen Eigenschaften.

Dies gilt für die Erbsubstanz DNA, insbesondere jedoch für die weniger stabile RNA, die nach Entnahme der biologischen Probe durch die ubiquitären RNAsen sehr schnell abgebaut werden kann. Dasselbe gilt für DNA-Methylierungsmuster, die durch Umwelteinflüsse nach Probenentnahme verloren gehen oder verfälscht werden können

Hinzu kommt, dass es nach der Entnahme einer biologischen Probe, z.B. eines Schnitts, einer Biopsie oder dergleichen - durch Induktion von Stressgenen und dergleichen auch zur Synthese neuer mRNA-Moleküle kommen kann, wodurch das Transkriptionsmuster der Zellen verändert werden kann.

Insbesondere im medizinischen Bereich ist die Stabilisierung von Nukleinsäuren notwendig, da hier häufig nukleinsäurehaltige Proben genommen werden, die erst nach längerer Lagerung und einem Transport in ein Labor weiter untersucht werden können. In der Zwischenzeit können sich die in den Proben enthaltenen Nukleinsäuren verändern oder sogar vollständig zersetzen. Dies beeinflusst das Ergebnis später durchgeführter Tests massiv oder macht diese gänzlich unmöglich. Ähnlich ungünstige Voraussetzungen finden sich z.B. in der Forensik oder in der Probennahme unter Feldbedingungen

Ebenso ist für die Untersuchung des Proteoms die Stabilisierung von Proteinen unbedingt notwendig, da Proteine sehr rasch durch Modifikation, wie z. B. Phosphorylierung und Dephosphorylierung verändert werden, und ebenso wie Nukleinsäuren spezifisch oder unspezifisch degradiert werden können, bzw. eine Neusynthese nach Induktion stattfinden kann.

Für die Stabilisierung von Nukleinsäuren und Proteinen in kompakten Gewebeproben ergibt sich im Vergleich zu anderen biologischen Proben noch eine weitere Schwierigkeit. Gewebe sind kompakt, vielschichtig und heterogen in Bezug auf die Zusammensetzung, die Inhaltsstoffe und den Aufbau. Für die Stabilisierung von Nukleinsäuren in Gewebeproben muss sich die Wirkung des stabilisierenden Reagenzes nicht nur an der Oberfläche von Zellen bzw. innerhalb einer Zellschicht entfalten, sondern auch tief innerhalb des kompakten, vielschichtigen Probenmaterials wirken. Zudem müssen in Bezug auf ihre Inhaltstoffe und Morphologie sehr verschiedene Gewebetypen adressiert werden können. Unterschiede finden sich dabei z.B. in Zellstruktur, Membranaufbau, Abgrenzungen / Kompartimentierungen und Inhaltsstoffen, insbesondere dem Protein-, Kohlenhydrat- oder Fettgehalt.

Die Stabilisierung soll dabei erfolgen, ohne dass die biologische Probe zerstört wird oder zur Stabilisierung zerstört werden muss. Insbesondere Gewebeproben, aber auch zelluläre Proben werden häufig zusätzlich zur molekularen Analyse auch zu einer morphologischen Untersuchung herangezogen. Diese soll nach Stabilisierung der Probe weiterhin möglich sein. Im Idealfall trägt eine zur Stabilisierung des Genoms, Transkriptoms, Proteoms und/oder Methyloms verwendete Substanz auch zur Stabilisierung und zum Erhalt der morphologischhistologischen Integrität bei.

Die Stabilisierung muss durch sehr einfaches und rasches Handling der Probe zu gewährleisten sein, da zum einen jegliche notwendige Vorbehandlung der Probe (wie z. B. Waschen oder Homogenisieren) die unverzügliche Stabilisierung des Genexpressionsprofils verhindert, da während der Verzögerung durch die Vorbehandlung z.B. RNA abgebaut oder neu synthetisiert werden kann. Zum anderen erschwert jegliche Vorbehandlung und jeder zusätzliche Prozessierungsschritt den Einsatz des Stabilisierungsmittels. Nur bei sehr einfacher Handhabung ohne notwendiges Gerät und weitere Probenvorbereitung ist ein Einsatz an jeglichem Ort, an dem biologische Proben anfallen, denkbar, wie. z. B. im Operationssaal, bei Freilanduntersuchungen, in einem Lebensmittel herstellenden Betrieb, an einem kriminologischen Tatort, und dergleichen.

### Stand der Technik

WO 2004/072228 offenbart ein Verfahren zur Behandlung von biologischen Proben, um diese für die Nukleinsäureextraktion vorzubereiten. Zu diesem Zweck wird die biologische Probe mit einem proteindenaturierendem Agenz behandelt und zwar in Kombination mit einem aprotischen Lösungsmittel, stufenweiser Erhitzung und/oder einer Probenverdünnung um die Proteine in der Probe zu denaturieren, die Zelllyse zu bewirken und die Nukleinsäuren aus den lysierten, d.h. zerstörten Zellen freizusetzen.

EP 0 578 885 offenbart ein Verfahren, bei dem ein zu transplantierendes Gewebe, wie bspw. eine Herzklappe, in einen festen Kunststoff (Polyurethan) eingebettet und damit fixiert wird, um es länger haltbar zu machen und ihm die nötige mechanische Stabilität zu verleihen. Um das Gewebe für die Behandlung mit dem Fixierungsmittel Polyurethan vorzubereiten, werden aprotische Lösungsmittel eingesetzt, um dem Gewebe Wasser zu entziehen.

Eine im Stand der Technik bekannte und sehr häufig verwendete Form der Stabilisierung von Gewebeproben inklusive aller Bestandteile ist das Einfrieren bzw. Tiefgefrieren der Proben. Hierbei wird die Probe direkt nach der Entnahme aus ihrer natürlichen Umgebung bei unter -80°C in flüssigem Stickstoff tiefgefroren. Die so behandelte Probe kann dann ohne Integritätsveränderungen bei etwa -70°C nahezu unbegrenzt gelagert werden. Derartige Verfahren erfordern jedoch durchweg sehr aufwendige, logistische Voraussetzungen, da ein Auftauen der Proben während des Transports, der Lagerung oder während der unterschiedlichsten An- bzw. Verwendungsprozesse verhindert werden muss. Neben den zusätzlichen Kosten für spezielle Probenaufnahmegefäße sowie für die permanente Kühlung der Proben, ist zudem der Einsatz von flüssigem Stickstoff nicht nur sehr umständlich, sondern auch nur unter besonderen Vorsichtsmaßnahmen durchführbar.

Darüber hinaus gestaltet sich eine nachfolgende Analyse des gefrorenen Probenmaterials, insbesondere einzelner Bestandteile der Probe, zumeist sehr schwierig. So führt beispielsweise das Auf- oder Antauen der Probe während der Lagerung, des Transportes oder der Verarbeitung zum Abbau insbesondere der RNA. D. h. derartig an- bzw. aufgetaute Proben liefern keine reproduzierbaren Ergebnisse mehr. Zudem sind gerade Gewebestücke im gefrorenen Zustand nur schwer manuell oder nur mit einem erhöhten apparativen Aufwand zu verarbeiten, beispielsweise zu zerteilen.

Ebenso ist die Stabilisierung mittels Formalin und anschließendes Einbetten der stabilisierten Proben, beispielsweise in Parafin, für histologische Gewebeuntersuchen bereits seit langem bekannt. Eine solche Stabilisierung ist gleichwohl zumeist ungeeignet für die Anwendung molekularbiologischer Verfahren, da nur eine sehr unzureichende Stabilisierung der Nukleinsäuren erfolgt, und so maximal ein qualitativer, nicht aber ein quantitativer Nachweis der vorhandenen Nukleinsäuren bzw. Nukleinsäurefragmente möglich ist. Darüber hinaus führt die Stabilisierung mit vernetzend wirkenden Stabilisierungsmitteln wie Formalin zu einer verminderten Extrahierbarkeit der Nukleinsäuren oder Proteine aus den Geweben. Auch ist Formalin aus toxikologischen Gründen nicht unbedenklich.

Konservierungssubstanzen, wie z. B. die in US 5,010,184, US 5,300,545, WO-A-02/00599 und WO-A-02/00600 beschriebenen kationische Detergentien, mit denen hingegen sehr gute qualitative Nachweise der Nukleinsäuren erfolgen können, eigenen sich nur für Proben, welche Einzelzellen enthalten bzw. nur eine Zellschicht aufweisen. Für eine Stabilisierung von Nukleinsäuren in kompakten Gewebestücken sind derartige Konservierungssubstanzen hingegen nicht ausreichend.

Darüber hinaus eigenen sich diejenigen Reagenzien und Verfahren, mit denen Nukleinsäuren für qualitative Nachweise stabilisiert werden können, in der Regel auch nicht zur gleichzeitigen Stabilisierung von Proteinen, da hier unterschiedliche biochemische Voraussetzungen gelten. Zudem kann mit derartig stabilisierten Proben keine histologische Untersuchung durchgeführt werden, da das Stabilisierungsmittel zwar z. B. die Nukleinsäuren, aber nicht die Zell- bzw. Gewebestrukturen konserviert.

Andere Konservierungssubstanzen, welche beispielsweise hochkonzentriertes Ammoniumsulfat (siehe z. B. US 6,204,375) enthalten, eignen sich gut zur Stabilisierung von Nukleinsäuren in unterschiedlichen Geweben. Sie sind jedoch weitgehend ungeeignet für den Einsatz zur Stabilisierung von zellhaltigen oder zellfreien Körperflüssigkeiten, wie z. B. Blut, Serum oder Plasma und weisen zudem weniger gute Stabilisierungseigenschaften bei einigen Gewebetypen, wie z. B. Fettgewebe, auf. Hinzu kommt, dass die strukturellen Eigenschaften z.B. einer Gewebeprobe durch die Ammoniumsulfat-Behandlung verloren gehen; letztere eignet sich also nicht für die Konservierung histologischer Proben.

Grundsätzlich können Arbeiten mit Zellen oder anderen biologischen Proben nicht unbedingt auf kompakte Gewebe übertragen werden. Für die Stabilisierung von Nukleinsäuren in kompakten Gewebeproben ergibt sich im Vergleich zu anderen biologischen Proben eine besondere Schwierigkeit. Gewebe sind, bezüglich ihrer Zusammensetzung, ihrer Inhaltsstoffe sowie dem Aufbau vielschichtig und heterogen. Für die Stabilisierung von Nukleinsäuren in kompakten Gewebeproben muss sich die Wirkung des stabilisierenden Reagenzes nicht nur an der Oberfläche der Zellen bzw. innerhalb einer Zellschicht entfalten, sondern auch tief innerhalb des vielschichtigen Probenmaterials wirken. Zudem müssen häufig innerhalb ein und derselben biologischen Probe sehr verschiedene Gewebe- und/oder Zelltypen adressiert werden können, die sich beispielsweise in der Zellstruktur, dem Membranaufbau, den Kompartimentierungen und den Biomolekülen, beispielsweise hinsichtlich der Proteine, der Kohlenhydrate und/oder dem Fettgehalt, unterscheiden.

Zur Verringerung der Nachteile beim Verarbeiten von gefrorenen Proben insbesondere für die Isolierung von RNA sind ebenfalls sogenannte Transitionslösungen beschrieben. Dabei wird zunächst das gefrorene Gewebe in eine auf -70°C bis -80°C vorgekühlte Lösung überführt und anschließend darin für einige Stunden (mindestens 16 Std.) bei etwa -20°C gelagert. Nachfolgend kann dann die mit der Transitionslösung durchtränkte Probe nur für einen kurzen Zeitraum, beispielsweise maximal zum Zerteilen der Probe, auf Arbeitstemperaturen von -4°C bis 0°C, ggf. auch bis zu Raumtemperatur, erwärmt werden, ohne dass sich der Nukleinsäurestatus der Probe verändert. Weitere Analysen sowie die Lagerung der Probe sind bei Raumtemperatur jedoch nicht möglich. Derartige, beispielsweise aus der WO-A-2004/72270 bekannte, Transitionslösungen bestehen vornehmlich aus einwertigen Alkoholen.

Nachteiligerweise bleiben die mit gängigen Transitionslösungen behandelten Proben nur für einen sehr kurzen Zeitraum bei Raumtemperatur stabil, womit die Zeit zur Verarbeitung nur knapp bemessen ist und insbesondere bei der Bearbeitung vieler Proben, insbesondere mit Schneide- und Wiegevorgängen, sehr leicht überschritten wird. Darüber hinaus erfolgt die Transition nur sehr langsam, wodurch keine direkten Experimente folgen können und sich Wartezeiten von zumeist einem Tag ergeben. Ebenso ist ein unbeschadeter Transport der so behandelten Proben bei Raumtemperatur unmöglich, da nicht nur die Transition bei Temperaturen von ≤ -20°C erfolgen muss, sondern auch die anschließende stabile Lagerung der Probe. Auch ein Transport der Probe ist nur bei ≤ -20°C möglich, was den Einsatz von Kühlmitteln, beispielsweise von Trockeneis, beim Transport erforderlich macht. Darüber hinaus ist zu beachten, dass die in der WO-A-2004/72270 eingesetzten einwertigen Alkohole, wie etwa Methanol, Ethanol oder Isopropanol, leichtentzündlich, flüchtig oder toxisch sind und somit bei deren Einsatz bestimmte Sicherheitsvorkehrungen zu treffen sind.

Durch die Verwendung der herkömmlichen Transitionslösungen werden zwar Verbesserungen beim Verarbeiten der Proben, wie z. B. dem Abwiegen oder Zuschneiden, erzielt, sie verringern jedoch weder den apparativen Aufwand (da die Lösung zur Transition bei -70 bis -80°C vorgekühlt werden muss und daher trotzdem eine entsprechende Kühlvorrichtung zur Verfügung stehen muss), noch können die mit der Transitionslösung behandelten Proben bei Raumtemperatur über einen längeren Zeitraum stabilisiert werden.

An all dem zeigt sich, dass es schwierig ist, in einer biologischen Proben einerseits gleichzeitig das Genom, das Transkriptom, das Proteom und das Methylom (also DNA, RNA, Proteine sowie das Methylierungsmuster), und andererseits auch den histologisch-morphologischen Zustand der Probe mit geringem Aufwand, zu stabilisieren. Um z.B. eine Probe vor einem RNA-Abbau durch die ubiquitären RNAsen zu schützen, wäre es sinnvoll, sämtliche Enzyme im Medium (und damit auch alle RNAsen) zu denaturieren. Dies würde jedoch dem oben gesteckten Ziel entgegenlaufen, da hierdurch das Proteom massiv beeinträchtigt würde. Dasselbe würde gelten, wenn man versuchen würde, Proteasen zu denaturrieren, um den proteasevermittelten Proteinabbau zu verhindern. Umgekehrt werden durch eine Maßnahme, die das Proteom einer Probe möglichst unbeeinträchtigt lässt, auch die ubiquitären RNAsen geschont, so dass eine Beeinträchtigung des Transkriptoms zu befürchten ist.

### Aufgabe der vorliegenden Erfindung

Der vorliegenden Erfindung liegt die Aufgabe zugrunde, die beschriebenen, sich aus dem Stand der Technik ergebenden Nachteile zu überwinden.

Darüber hinaus liegt der vorliegenden Erfindung die Aufgabe zugrunde, ein Verfahren zur Stabilisierung einer biologischen Probe anzugeben, mit dem sowohl gefrorene als auch frische biologische Proben bei möglichst moderaten Temperaturbedingungen, beispielsweise auch bei Raumtemperatur, ohne Beeinträchtigung des Genoms, des Transkriptoms, des Proteoms, des Metaboloms und des Methyloms bzw. des histologisch-morphologischen Zustands der biologischen Probe stabilisiert werden können.

Darüber hinaus soll das Verfahren zur Stabilisierung einer biologischen Probe auch eine Analyse der in der biologischen Probe enthaltenen Biomoleküle ermöglichen. In diesem Zusammenhang soll das Stabilisierungsverfahren es insbesondere ermöglichen, dass sowohl Proteine als auch Nukleinsäuren qualitativ und quantitativ in der stabilisierten biologischen Probe analysiert werden können. Zudem sollte durch die Stabilisierung der biologischen Probe die Qualität der Nukleinsäuren, welche beispielsweise durch Gel-Analyse oder durch die Anzahl der PCR-Zyklen bis zum Erreichen einer bestimmten Nukleinsäuremenge bestimmt werden kann, und die Qualität der Proteine, welche beispielsweise im Falle eines Enzyms durch Western-Blot-Analysen und ggf. entsprechende Aktivitätstests bestimmt werden kann, nicht oder allenfalls nur geringfügig beeinträchtigt werden.

Weiterhin soll das Verfahren zur Stabilisierung einer biologischen Probe zu einer stabilisierten biologischen Probe führen, die nicht nur bei moderaten Temperaturen, beispielsweise bei Raumtemperatur, analysiert werden kann, sondern gegebenenfalls vor oder nach einer solchen Analyse für möglichst lange Zeit bei solchen moderaten Temperaturbedingungen gelagert werden kann.

Im Falle von Biomolekülen soll dabei unter dem Begriff "Stabilisierung" vorzugsweise die Hemmung des Abbaus, der Modifikation, der Induktion oder der Änderung der Aktivität von Biomolekülen verstanden werden. Im Falle von histologischen Analysen der biologischen Proben soll unter dem Begriff "Stabilisierung" vorzugsweise das Verhindern einer wesentlichen Änderung der Morphologie der Proben verstanden werden.

### Zusammenfassung der Erfindung

Diese Aufgabe wird mit den Merkmalen des vorgelegten Hauptanspruchs gelöst. Die Unteransprüche geben bevorzugte Ausführungsformen an. Dabei ist zu beachten, dass die genannten Bereichsangaben durchweg einschließlich der jeweiligen Grenzwerte zu verstehen sind.

Demgemäß ist vorgesehen, ein Verfahren zur Stabilisierung von Zellen und Biomolekülen in einer biologischen Probe bereit zu stellen, wobei die Stabilisierung ohne die Zerstörung der biologischen Probe erfolgt, welches die folgenden Schritte aufweist:
a) Bereitstellen einer biologischen Probe enthaltend Zellen, und
b) in Kontakt bringen der biologischen Probe mit einer Zusammensetzung, aufweisend eine Substanz gemäß der folgenden Strukturformel: 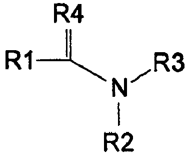 worin R1 ein Wasserstoffrest oder ein Methylrest ist, R2 und R3 gleiche oder unterschiedliche Kohlenwasserstoff-Reste mit einer Kohlenstoff-Kettenlänge von 1 - 20 sind, sowie R4 ein Sauerstoff-, ein Schwefel- oder ein Selenrest ist,
   wobei es sich bei der biologischen Probe enthaltend Zellen um ein Material ausgewählt aus Körperflüssigkeiten, Blut, Leukozytenfraktionen, Crusta Phlogistica, Sputum, Speichel, Urin, Sperma, Faeces, Abstriche, Suspensionen, flüssige Proben, Plasma, Serum und Zellpellets handelt und wobei die Stabilisierung die Isolierung von Biomolekülen aus der stabilisierten Probe erlaubt.

Die Kohlenwasserstoffreste R2 und/oder R3 können dabei unabhängig voneinander ausgewählt sein aus der Gruppe enthaltend Alkyl, langkettige Alkyl, Alkenyl, Alkoxy, langkettige Alkoxy, Cycloalkyl, Aryl, Halogenalkyl, Alkylsilyl, Alkylsilyloxy, Alkylen, Alkendiyl, Arylen, Carboxylate und Carbonyl. Innerhalb der Beschreibung und den Ansprüchen werden allgemeine Gruppen, wie z.B: Alkyl, Alkoxy, Aryl etc. beansprucht und beschrieben. Wenn nicht anders beschrieben, werden bevorzugt die folgenden Gruppen innerhalb der allgemein beschriebenen Gruppen im Rahmen der vorliegenden Erfindung verwendet:
- Alkyl: lineare und verzweigte C1-C5-Alkyle,
- langkettige Alkyle: lineare und verzweigte C5-C20 Alkyle
- Alkenyl: C2-C6-Alkenyl,
- Cycloalkyl: C3-C8-Cycloalkyl,
- Alkoxy: C1-C6-Alkoxy,
- langkettig Alkoxy: lineare und verzweigte C5-C20 Alkoxy,
- Alkylene: ein zweiwertiger linearer oder verzweigter aliphatischer, zykloaliphatischer oder aromatischer gegebenenfalls Heteroatome enthaltender Kohlenwasserstoffrest mit 2 bis 18 Kohlenstoffatomen ist, z.B. ausgewählt aus der Gruppe enthaltend: Methylen; 1,1-Ethylen; 1,2-Ethylen; 1,1-Propyliden; 1,2-Propylen; 1,3- Propylen; 2,2-Propyliden; Butan-2-ol-1,4-diyl; Propan-2-ol-1,3-diyl; 1,4-Butylen; 1,4-Pentylen, 1,6-Hexylen, 1,7-Heptylen, 1,8-Octylen, 1,9-Nonylen, 1,10-Decylen, 1,11-Undecylen, 1,12-Docedylen, Cyclohexan-1,1-diyl; Cyclohexan-1,2-diyl; Cyclohexan-1,3-diyl; Cyclohexan-1,4-diyl; Cyclopentane-1,1-diyl; Cyclopentan-1,2-diyl; and Cyclopentan-1,3-diyl
- Alkendiyl: ausgewählt aus der Gruppe enthaltend: 1,2-Propendiyl, 1,2-Butendiyl, 2,3-Butendiyl, 1,2-Pentendiyl, 2,3-Pentendiyl, 1,2-Hexendiyl, 2,3-Hexendiyl, 3,4-Hexendiyl
- Alkindiyl: gleich -C≡C- ist,
- Aryl: ausgewählt aus Aromaten mit einem Molekulargewicht unter 300 Da,
- Arylene: ausgewählt aus der Gruppe enthaltend: 1,2-Phenylen; 1,3- Phenylen; 1,4-Phenylen; 1,2-Naphtalenylen; 1,3-Naphtalenylen; 1,4- Naphtalenylen; 2,3-Naphtalenylen; 1-Hydroxy-2,3-phenylen; 1-Hydroxy-2,4- phenylen; 1-Hydroxy-2,5- phenylen; and 1-Hydroxy-2,6-phenylen,
- Carboxylat: die Gruppe -C(O)OR, wobei R ausgewählt ist aus: Wasserstoff; C1-C6-Alkyl; Phenyl; C1-C6-Alkyl-C6H5; Li; Na; K; Cs; Mg; and Ca,
- Carbonyl: die Gruppe -C(O)R, wobei R ausgewählt ist aus: Wasserstoff; C1-C6-Alkyl; Phenyl; C1-C6-Alkyl-C6H5 and Amin (resultierend in einem Amid) ausgewählt aus der Gruppe: -NR'2, wobei jedes R' unabhängig ausgewählt ist aus: Wasserstoff; C1-C6-Alkyl; C1-C6-Alkyl-C6H5; und Phenyl, wobei, wenn beide R C1-C6 Alkyl darstellen, diese einen NC3 bis NC5 heterocyclischen Ring bilden können, wobei die übrige Alkylkette Alkylsubsituenten des Rings bilden,
- Alkylsilyl: die Gruppe -SiR₁R₂R₃, wobei R₁, R₂ und R₃ unabhängig voneinander ausgewählt sind aus: Wasserstoff; Alkyl; langkettiges Alkyl, Phenyl, Cycloalkyl, Halogenalkyl, Alkoxy, langkettiges Alkoxy,
- Alkylsilyloxy: die Gruppe -O-SiR₁R₂R₃, wobei R₁, R₂ und R₃ unabhängig voneinander ausgewählt sind aus: Wasserstoff; Alkyl; langkettiges Alkyl, Phenyl, Cycloalkyl, Halogenalkyl, Alkoxy, langkettiges Alkoxy.

Festzuhalten bleibt jedoch, dass die Kohlenwasserstoffreste R2 und/oder R3 besonders bevorzugt Alkylreste und/oder langkettige Alkylreste gemäß der obigen Definition sind.

Dabei hat die Kettenlänge dieser Kohlenwasserstoffreste verschiedene Einflüsse, die im Folgenden diskutiert werden sollen.

Zunächst einmal hat die Kettenlänge einen Einfluss auf die Permeation der erfindungsgemäßen Zusammensetzung in die Probe; bei großen Kettenlängen wird die Permeation aus sterischen Gründen verzögert; allerdings gibt es Anzeichen darauf, dass der Konservierungseffekt von Zusammensetzungen mit großen Kettenlängen nachhaltiger ist.

Bei in Suspension vorliegenden Proben (z.B. Blutproben, Urin, Abstriche etc.) ist das Problem der Permeation weniger akut, da hier die Diffusionswege weitaus geringer sind, und so auch erfindungsgemäße Substanzen mit sehr großen Kettenlängen an R2 und/oder R3 ausreichend schnell zu allen zu konservierenden Bereichen vordringen.

Hinzu kommt, dass der erfindungsgemäßen Zusammensetzung bei sehr großen Kettenlängen an R2 und/oder R3, die dazu führten, dass die Substanz als Feststoff vorliegt, ein Lösungsmittel hinzugefügt werden kann, um die Substanz zu lösen und so für die Konservierung der Probe zugänglich zu machen.

Alternativ kann die Substanz auch als Feststoff eingesetzt und direkt in einer flüssigen Probe gelöst werden.

Sehr große Kettenlängen an R2 und/oder R3 führen andererseits ggf. zu einer größeren Hydrophobizität der erfindungsgemäßen Zusammensetzung und können z.B. die Permeation - und damit die Konservierung - von fett- oder lipidreichen Proben begünstigen; bei solchen Proben weisen hingegen erfindungsgemäße Zusammensetzungen mit geringen Kettenlängen an R2 und/oder R3 u.U. Permeationsprobleme auf.

Der Fachmann wird aufbauend auf dem oben gesagten entsprechend der jeweiligen Bedürfnisse die Kettenlängen an R2 und/oder R3 gezielt wählen, ohne selbst erfinderisch tätig zu werden. Allenfalls in Routineexperimente erforderlich, um Löseverhalten, Permeation und Konservierungseignung der jeweils ausgewählten Substanzen zu bestimmen. Gerade in Bezug auf die Konservierungseignung findet der Fachmann in den Beispielen der vorliegenden Erfindung umfassende Hinweise darauf, wie diese Eigenschaft untersucht werden kann.

Dabei kann die Kettenlänge n an R2 und/oder R3 insbesondere die Werte 1, 2, 3, 4, 5, 6, 7, 8, 9, 10, 11, 12, 13, 14, 15, 16, 17, 18, 19 und 20 annehmen.

Bevorzugt ist vorgesehen, dass R2 und R3 Kohlenstoff-Kettenlängen von 1 - 10 aufweisen. Hierbei kann die Kettenlänge n insbesondere die Werte 1, 2, 3, 4, 5, 6, 7, 8, 9, und 10 annehmen.

Besonders bevorzugt ist vorgesehen, dass R2 und R3 Kohlenstoff-Kettenlängen von 1 - 5 aufweisen. Hierbei kann die Kettenlänge n insbesondere die Werte 1, 2, 3, 4 und 5 annehmen.

Besonders bevorzugt werden an R2 und/oder R3 jedoch insbesondere Methyl- und Ethylreste eingesetzt. Die Kettenlänge n nimmt hier also Werte von 1 und 2 an.

Grundsätzlich ist anzumerken, dass die in der erfindungsgemäßen Zusammensetzung verwendete Substanz als alleiniges Agens zur Konservierung verwendet werden kann. Ebenso kann die Substanz im Zusammenspiel mit anderen Konservierungssubstanzen oder sogar nur als Additiv zu anderen Konservierungssubstanzen in der Zusammensetzung verwendet werden. Konservierungssubstanzen finden im Text Erwähnung.

Dabei kann das Volumen- bzw. Gewichtsverhältnis zwischen der erfindungsgemäßen Substanz und einer oder mehrerer anderer Konservierungssubstanzen in der erfindungsgemäßen Zusammensetzung im Bereich zwischen 0,01 : 100 und 100 : 0 liegen. Bevorzugt liegt es im Bereich zwischen 0,1 : 100 und 100 : 0 und besonders bevorzugt liegt es im Bereich zwischen 1 : 100 und 100 : 0 und insbesondere bevorzugt liegt es im Bereich zwischen 5 : 100 und 100: 0.

In Bezug auf das Volumen- bzw. Gewichtsverhältnis zwischen erfindungsgemäßer Zusammensetzung und zu konservierender Probe müssen verschiedene Fälle unterschieden werden.

Für den Fall, dass die erfindungsgemäße Zusammensetzung in flüssiger Form und die zu konservierende Probe in fester Form vorliegt, ist z.B. vorgesehen, dass 0,1 -1 ml erfindungsgemäßer Zusammensetzung mit 100 mg Probe versetzt werden. Es gibt dabei kein technisch begründetes maximales Zugabevolumen, welches daher eher von praktischen Erwägungen (z. B. Gefäßgröße) bestimmt wird.

Für den Fall hingegen, dass die erfindungsgemäße Zusammensetzung in fester/kristalliner Form und die zu konservierende Probe in flüssiger Form (z.B. Blutprobe) vorliegt, können weit kleinere Volumen- bzw. Gewichtsverhältnisse verwendet werden; diese liegen z.B. im Bereich zwischen 5 : 1 und 1 : 1000.

Der Anteil der erforderlichen erfindungsgemäßen Zusammensetzung ist natürlich auch von der Menge des zu konservierenden Materials abhängig. Da z.B. eine Urinprobe überwiegend Wasser enthält, das nicht konserviert werden muss, kann der Anteil der erfindungsgemäßen Zusammensetzung sehr viel geringer ausfallen, als wenn z.B. eine viel zelluläres Material enthaltende Probe konserviert werden soll.

Bei der erfindungsgemäß verwendeten Stoffklasse handelt es sich z.B. um Dialkylacetamide (wenn R1 ein Methylrest ist) bzw. Dialkylformamide (wenn R1 ein Wasserstoffrest ist.). Bei diesen Stoffen handelt es sich um polare Lösungsmittel, die insbesondere in der Kunststoff- und Fasertechnik zum Einsatz kommen. Im Zusammenhang mit biologischen Anwendungen ist hingegen nur die Verwendung von N,N-Dimethylacetamid zur Induktion der Differenzierung von Erythroid-Zellen bekannt (M Tanaka et al. (1975), "Induction of erythroid differentiation in murine virus infected eythroleukemia cells by highly polar compounds." PNAS, 72, 1003), nicht jedoch die Eignung dieser Stoffe zur Stabilisierung von biologischen Proben.

Es ist das Verdienst der Erfinder, erstmals erkannt zu haben, dass sich diese Stoffe auch für die Stabilisierung von biologischen Proben eignen. Diese Erkenntnis ist umso überraschender, als die bisherigen Anwendungsbereiche dieser Stoffe eine solche Verwendbarkeit eher unwahrscheinlich haben erscheinen lassen.

Über den Wirkmechanismus in Bezug auf die Probenkonservierung können die Erfinder bislang lediglich spekulieren. Es darf jedoch aufgrund experimenteller Befunde als sicher gelten, dass, obwohl insbesondere die unter die obige Definition fallenden Dialkylformamide strukturelle Ähnlichkeiten mit den Fixativen Formaldehyd und Acetaldehyd aufweisen, anders als bei den letztgenannten keine Fixierung bzw. Quervemetzung der Makromoleküle stattfindet.

Bevorzugt ist dabei vorgesehen, dass es sich bei der biologischen Probe um eine gefrorene biologische Probe handelt. Solche Proben stehen z.B. in Gen- und Gewebebanken in großer Zahl zur Verfügung; deren weitere Analyse würde von dem erfindungsgemäßen Verfahren enorm profitieren. Dies ist insbesondere für die epidemiologische Analyse von älteren Proben interessant, aber auch für die Forensik, die Archäologie und dergleichen. Ebenso kann es sich bei der Probe je-doch auch um eine nicht-gefrorene biologische Probe handeln. Hier kommen die Vorteile der Erfindung besonders zum Tragen, da auf aufwendige Kühlausrüstung verzichtet werden kann, um eine Probe unmittelbar nach deren Gewinnung zu stabilisieren. Auch dies ist für die eingangs genannten Anwendungsbereiche von großem Vorteil.

Besonders bevorzugt handelt es sich bei der biologischen Probe um ein Material ausgewählt aus der Gruppe enthaltend Probenmaterial, Plasma, Körperflüssigkeiten, Blut, Serum, Zellen, Leukozytenfraktionen, Crusta Phlogistica, Sputum, Speichel, Urin, Sperma, Faeces, und Abstriche.

Bei der erfindungsgemäß verwendeten Substanz handelt es sich bevorzugt um eine Substanz ausgewählt aus der Gruppe enthaltend N,N-Dimethylacetamid, N,N-Diethylacetamid, N,N-Dimethylformamid, N,N-Diethylformamid, N,N-Dimethylthioforamid und N,N-Diethylthioforamid. Deren Strukturformeln lauten wie folgt:

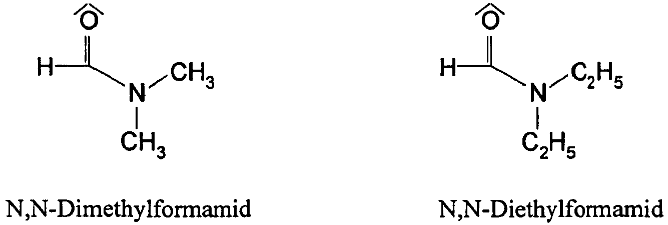

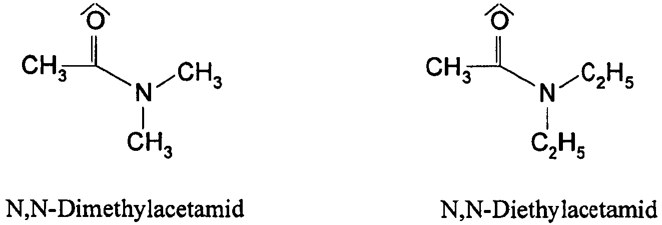

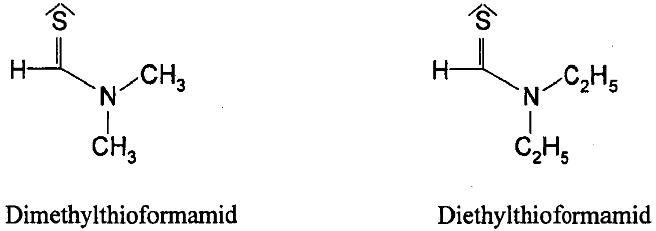

Weiterhin ist erfindungsgemäß vorgesehen, dass die Probe außerdem mit einem oder mehreren Stoffen in Kontakt gebracht wird, ausgewählt aus der Gruppe Lösemittel, Fixative, Puffersubstanzen, Chelatoren, Mittel zur Verbesserung der Sichtbarkeit des Zellkern, quervernetzende Stoffe wie Formaldehyd, p-Formaldehyd, Acetaldehyd oder Glutaraldehyd.

Besonders bevorzugte Stoffe sind hier insbesondere organische Säuren wie z.B. Essigsäure und DMSO.

In einer bevorzugten Ausgestaltung ist vorgesehen, dass das in Kontakt bringen der biologischen Probe mit der Zusammensetzung bei einer Temperatur von 0°C, 8°C, 18°C oder 30°C erfolgt.

Dabei bedeutet die Formulierung, dass das "in Kontakt bringen der biologischen Probe mit der Zusammensetzung bei einer der vorstehend genannten Temperaturen erfolgt, dass nach dem in Kontakt bringen der biologischen Probe mit der Zusammensetzung die Temperatur der auf diese Weise erhaltenen Mischung innerhalb der vorstehend genannten Temperaturen liegt. So kann es beispielsweise sein, dass als biologisches Material eine auf Temperaturen von weniger als -20°C tiefgefrorene Probe, beispielsweise eine in flüssigem Stickstoff gelagerte Probe eingesetzt wird, wobei in diesem Falle eine solche Menge an Zusammensetzung bzw. eine Zusammensetzung mit einer solchen Temperatur eingesetzt wird, dass nach dem in Kontakt bringen der tiefgefrorenen biologischen Probe mit der Zusammensetzung die Temperatur der Mischung (und somit auch die Temperatur der biologische Probe) im vorstehend genannten Temperaturbereich liegt.

Weiterhin kann es gemäß einer besonderen Ausführungsform des erfindungsgemäßen Verfahrens auch bevorzugt sein, dass die biologische Probe nach dem in Kontakt bringen mit der Zusammensetzung, vorzugsweise unter den vorstehend genannten Temperaturbedingungen, anschließend bei Raumtemperatur gelagert wird, wobei diese Lagerung über einen Zeitraum von mindestens einem Tag, vorzugsweise mindestens 2 Tagen, darüber hinaus bevorzugt mindestens 3 Tagen, gegebenenfalls für mindestens eine Woche, mindestens zwei Wochen, mindestens einen Monat, mindestens drei Monate, mindestens sechs Monate oder auch mindestens 12 Monate erfolgen kann.

Das Verfahren gemäß der vorliegenden Erfindung ermöglicht die Lagerung einer behandelten biologischen Probe bei Raumtemperatur, bei Kühlschranktemperaturen oder bei noch höheren Temperaturen, ohne dass es zu einem erkennbaren Abbau von Biomolekülen wie Nukleinsäuren, Proteinen oder Metaboliten in der biologische Probe kommt. Dieses stellt einen signifikanten Vorteil gegenüber herkömmlichen Stabilisierungsverfahren dar, da das Verfahren ohne den Einsatz von flüssigem Stickstoff oder von Tiefkühlvorrichtungen durchgeführt und die stabilisierte Probe auch ohne den Einsatz von flüssigem Stickstoff oder von Tiefkühlvorrichtungen gelagert werden kann. Insbesondere in Gegenden mit mangelnder Infrastruktur ist dies von besonderem Vorteil, und macht das Verfahren daher z.B. für epidemiologische Untersuchungen in Entwicklungsländern, für paläontologische und archäologische Untersuchungen, für Untersuchungen im Feld oder für die Forensik interessant.

Nach der erfindungsgemäßen Behandlung und gegebenenfalls vor oder auch nach einem möglichen Lagerungsschritt kann die behandelte biologische Probe auch in geeignete Einbettungsmittel eingebettet werden, beispielsweise in Paraffin oder dergleichen.

Weiterhin kann es gemäß einer besonderen Ausführungsform des erfindungsgemäßen Verfahrens bevorzugt sein, dass sich an die vorgenannten Verfahrensschritte noch der Schritt einer histologisch-morphologischen Analyse der mit der Zusammensetzung in Kontakt gebrachten biologischen Probe und/oder der Schritt einer Analyse von Biomolekülen in der oder aus der mit der Zusammensetzung in Kontakt gebrachten biologischen Probe anschließt, wobei dieser Verfahrensschritt gegebenenfalls auch vor oder nach einer Lagerung gemäß dem vorstehend beschriebenen Verfahrensschritt durchgeführt werden kann.

Unter einer histologischen Analyse wird vorzugsweise jedes Untersuchungsverfahren verstanden, welches geeignet ist, den morphologischen Zustand einer Zelle oder von subzellulären Strukturen zu analysieren, beispielsweise mittels Mikroskopie und gegebenenfalls unter Einsatz von dem Fachmann bekannten Färbe- oder Markierungstechniken.

Für die histologisch-morphologische Analyse kann insbesondere vorgesehen sein, dass die Probe vor der Analyse mit geeigneten Farbstoffen oder mit gelabelten Antikörpern gegen bestimmte Probenbestandteile, wie z.B. Tumormarker oder dergleichen mehr, versetzt wird. Die Analyse findet z.B. mit Licht-, Fluoreszenz- oder Videomikroskopie statt. Die hierfür verwendeten histologisch-morphologischen Methoden, insbesondere Schneid-, Markierungs- und Färbetechniken sowie immunhistochemische Techniken, sind dem Fachmann aus der einschlägigen Literatur bekannt bzw. leicht auffindbar.

Bei der Analyse von Biomolekülen kann eine genomische, proteomische, metabolomische, transkriptomische oder methylomische Analyse vorgesehen sein. Die hierfür jeweils verwendeten molekularbiologischen Methoden, insbesondere Amplifikations-, Sequenzierungs und Nachweistechniken, sind dem Fachmann aus der einschlägigen Literatur bekannt bzw. leicht auffindbar.

Als Biomoleküle, die analysiert werden können, kommen alle dem Fachmann bekannten Biomoleküle in Betracht, insbesondere natürliche, modifizierte oder synthetische Nukleinsäuren, natürliche, modifizierte oder synthetische Proteine oder Oligopeptide, Hormone, Wachstumsfaktoren, Substrate des Stoffwechsels, Lipide, Oligosaccharide oder Proteoglukane. Als Nukleinsäuren kommen alle dem Fachmann bekannten Nukleinsäuren in Betracht, insbesondere Ribonukleinsäuren (RNA), beispielsweise mRNA, siRNA, miRNA, snRNA, t-RNA, hnRNA oder Ribozyme, oder Deoxyribonukleinsäuren (DNA). Grundsätzlich kann es sich um jeden Typ von Polynukleotid handeln, der ein N-Glykosid oder C-Glykosid einer Purin- oder Pyrimidinbase darstellt. Die Nukleinsäure kann einzel-, doppel- oder mehrsträngig, linear, verzweigt oder zirkulär sein. Sie kann einem in einer Zelle vorkommenden Molekül entsprechen, wie etwa genomische DNA oder Boten-RNA (mRNA), oder *in vitro* erzeugt werden wie Komplementär-DNA (cDNA), Gegenstrang-RNA (aRNA), oder synthetische Nukleinsäuren. Die Nukleinsäure kann aus wenigen Untereinheiten, mindestens zwei Untereinheiten, bevorzugt acht oder mehr Untereinheiten bestehen, wie etwa Oligonukleotide, mehreren hundert Untereinheiten bis hin zu mehreren tausend Untereinheiten, wie etwa bestimmte Expressionsvektoren, oder bedeutend mehr Untereinheiten, wie genomische DNA. Bevorzugt enthält die Nukleinsäure die kodierende Information für ein Polypeptid in funktionellem Zusammenhang mit regulatorischen Sequenzen, die die Expression des Polypeptids in der Zelle erlauben, in die die Nukleinsäure eingebracht wird oder in der sie natürlich vorliegt. So ist die Nukleinsäure in einer bevorzugten Ausfiihrungsform ein Expressionsvektor. In einer anderen Ausführungsform ist sie eine pDNA (Plasmid-DNA), eine siRNA, eine siRNA-Duplizes oder eine siRNA-hetero-Duplizes, wobei unter dem Begriff "siRNA" Ribonukleinsäuren mit einer Länge von etwa 22 Nukleotiden verstanden werden, die durch Spaltung einer doppelsträngigen RNA (dsRNA) durch das Enzym "Dicer" entstehen und in den Enzymkomplex "RISC" (RNA-induced silencing complex) eingebaut werden. Weitere Biomoleküle gemäß obiger Definition umfassen insbesondere Metabolite und Stoffwechselprodukte.

Die Formulierung "Analyse von Biomolekülen in der oder aus der mit der Zusammensetzung in Kontakt gebrachten biologischen Probe" bedeutet dabei, dass die Analyse sowohl *in situ* als auch *ex situ*, also beispielsweise nach Isolierung der Biomoleküle aus der biologischen Probe erfolgen kann. Sollen Biomoleküle aus einer biologischen Probe zum Zwecke der Analyse isoliert werden, so kann es vorteilhaft sein, insbesondere im Falle von Zellen, oder komplexen oder kompakten Proben die Proben zunächst zu homogenisieren, wobei dieses Homogenisieren auf mechanischem Weg, beispielsweise mittels Kanülen, Mörsern, Rotor-Stator-Homogenisatoren, einer Kugelmühle oder dergleichen, auf chemischem Weg durch den Einsatz geeigneter Lysepuffer, welche üblicherweise Detergenzien und/oder chaotrope Substanzen beinhalten, auf enzymatischem Weg, beispielsweise unter Einsatz von Proteasen, oder durch eine Kombination dieser Maßnahmen, durchgeführt werden kann.

Zur histologischen Analyse oder zur Analyse von Biomolekülen in der oder aus der biologischen Probe können dabei alle dem Fachmann bekannten und geeignet erscheinenden Analyseverfahren eingesetzt werden, vorzugsweise Verfahren ausgewählt aus der Gruppe umfassend die Lichtmikroskopie, die Elektronenmikroskopie, die konfokale Laserscanningmikroskopie, die Laser-Micro-Dissection, Scanningelektronenmikroskopie, das Western-Blotting, den Southern-Blotting, den Enzyme-linked Immonosorbent-Assay (ELISA), die Immunpräzipitation, die Affinitätschromatographie, die Mutationsanalyse, die Polyacrylamidgelelektrophorese (PAGE), insbesondere die zweidimensionale PAGE, die HPLC, die Polymerasekettenreaktion (PCR), die RFLP-Analyse (Restriction Fragment Length Polymorphism-Analyse), die SAGE-Analyse (Serial Analysis of Gen Expression), die FPLC-Analyse (Fast Protein Liquid Chromatography), die Massenspektrometrie, beispielsweise die MALDI-TOFF-Massenspektrometrie oder die SELDI-Massenspektrometrie, die Microarray-Analyse, die LiquiChip-Analyse, die Analyse der Aktivität von Enzymen, HLA-Typing, Sequenzierung, WGA ("Whole Genome Amplification"), WTA ("Whole Transcriptome Amplification"), RT-PCR, Real-Time-PCR bzw -RT-PCR, RNase-Protection-Analyse oder Primer-Extension-Analyse.

Dabei ist, wie bereits erwähnt, die Kombination von histologisch-morphologischen Befunden mit Befunden aus der molekularbiologischen Analyse für einige Einsatzszenarien, wie z.B. die Tumordiagnostik oder die Diagnostik neurodegenerativer Erkrankungen, besonders erfolgversprechend. Gemäß einer besonderen Ausführungsform des erfindungsgemäßen Verfahrens umfasst daher der betreffende Verfahrensschritt sowohl eine histologische Analyse der biologischen Probe als auch eine Analyse von Biomolekülen in der oder aus der biologischen Probe. Gemäß einer weiteren besonderen Ausführungsform des erfindungsgemäßen Verfahrens umfasst der betreffende Verfahrensschritt sowohl eine Analyse von Nukleinsäuren in der oder aus der biologischen Probe als auch eine Analyse von Proteinen in der oder aus der biologischen Probe.

Ferner ist erfindungsgemäß die Verwendung eines Kit of Parts in dem erfindungsgemäßen Verfahren vorgesehen, aufweisend die erfindungsgemäße Zusammensetzung. Bevorzugt kann besagter Kit auch ein ggf. verschließbares Gefäß zur Probenaufnahme, -aufbereitung und/oder -lagerung, und/oder Reagenzien zur Analyse von Biomolekülen in oder aus einer biologischen Probe oder zur Analyse der Morphologie einer biologischen Probe enthalten. Als Reagenzien zur Analyse von Biomolekülen kommen hier insbesondere Reagenzien zur Detektion und Quantifizierung von DNA, RNA, Proteinen und Methylierten Nucleotiden in Frage. Der Fachmann kann solche Reagenzien aus der Fachliteratur ohne eigene erfinderische Leistung auffinden. Häufig sind solche Reagenzien als Kits für die jeweils zu analysierenden Biomoleküle bereits fertig erhältlich. Diese Reagenzien umfassen insbesondere Farbstoffe zum Färben von Zellen oder Zellbestandteilen, Antikörper, gegebenenfalls markiert mit Fluoreszenzfarbstoffen oder Enzymen, eine Absorptionsmatrix, wie etwa DEAE-Zellulose oder eine Silica-Membran, Substrate für Enzyme, Agarose-Gele, Polyacrylamid-Gele, Lösungsmittel wie Ethanol oder Phenol, wässrige Pufferlösungen, RNase-freies Wasser, Lyse-Reagenzien, alkoholische Lösungen und dergleichen.

Dabei kann die Zusammensetzung bereits in einem Probensammelgefäß abgefüllt sein. Denkbar ist aber auch, dass das Kit als einen weiteren Bestandteil eine Dosierungsvorrichtung umfasst, die mit der Zusammensetzung gefüllt ist und mittels derer definierte Portionen der Zusammensetzung, vorzugsweise unter sterilen Bedingungen, in das Gefäß eingefüllt werden können. Eine solche Dosierungsvorrichtung kann beispielsweise in Form eines Seifenspenders ausgebildet sein.

Weiterhin ist erfindungsgemäß die Verwendung einer der vorstehend beschriebenen Zusammensetzungen bzw. des Kits in dem erfindungsgemäßen Verfahren. Ebenso ist erfindungsgemäß ein Verfahren zur Analyse einer biologischen Probe vorgesehen, wobei ein wie vorstehend beschriebenes Kit oder eine ein wie vorstehend beschriebene Zusammensetzung verwendet wird.

Die vorliegende Erfindung wird durch die im Folgenden gezeigten und diskutierten Beispiele sowie Abbildungen genauer erläutert. Dabei ist zu beachten, dass die

Beispiele nur beschreibenden Charakter haben und nicht dazu gedacht sind, die Erfindung in irgendeiner Form einzuschränken.

### Vergleichsbeispiel 1: Transition mit verschiedenen erfindungsgemäßen Lösungen

Lebergewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit verschiedenen, nicht verdünnten und nicht-gekühlen Lösungen (s. Tabelle 1) versetzt und für 3 Tage bei 2-8°C im Kühlschrank sowie für einen Tag und gegebenenfalls 3 Tage bei Raumtemperatur gelagert. Im Anschluss an die Transition wird das Gewebe aus der Transitionslösung entfernt. Zur RNA-Isolierung werden je 10 mg Gewebe 350 µl eines handelsüblichen Guanidinium-Isothiocyanat-Puffers, wie z. B. RLT-Puffer der Firma QIAGEN zugeben. Die Probe wird mit Hilfe einer Kugelmühle, wie z. B. MM300 der Firma QIAGEN, über einen Zeitraum von 5 min bei 20 Hz mit einer 5 mm Stahlkugel homogenisiert, wobei der Guanidinium-Isothiocyanat-Puffer auf aus dem Stand der Technik bekannte Weise die Zellen lysiert und die freigesetzten Proteine denaturiert. Anschließend werden die Lysate bei 14000 rpm für 3 min zentrifugiert. Vom Überstand werden zwei Portionen von je 350 µl, die entsprechend 10 mg Gewebe repräsentieren, abgenommen. Zu diesen Proben wird 1 Volumen (350 µl) 70 %-iges Ethanol zugefügt und durch mehrmaliges Auf- und Abpipettieren oder durch Vortexen über einen Zeitraum von ca. 5 s gemischt. Das Lysat wird anschließend in eine handelsübliche Silicamembran enthaltene spin-Säule, wie z. B. RNeasy-Säulen der Firma QIAGEN, aufgetragen und durch Zentrifugation (1 min bei 10000 xg) durch die Membran hindurchgeführt. Die RNA bleibt an der Membran gebunden und wird anschließend mit einem ersten handelsüblichen Guanidinium-Isothiocyanat-haltigen Waschpuffer, beispielsweise mit dem Puffer RW1 der Firma QIAGEN, und danach mit einem zweiten Tris-haltigen bzw. Tris- und alkoholhaltigen Waschpuffer, z. B. Puffer RPE der Firma QIAGEN, gewaschen. Dabei werden die Waschpuffer jeweils durch Zentrifugation (1 min bei 10000 xg) durch die Membran hindurchgeführt. Die Waschung mit dem zweiten Tris-haltigen bzw. Tris- und alkoholhaltigen Waschpuffer wird mit einem geringeren Volumen wiederholt wobei gleichzeitig die Membran durch die Zentrifugation (2 min max. rpm, hier 20000 x g) getrocknet wird. Zur Elution werden 40 µl RNase-freies Wasser auf die Membran pipettiert, um die gereinigte RNA von der Membran abzulösen. Nach einer Inkubation von 1 Minute bei einer Temperatur im Bereich von 10 - 30 °C wird das Eluat durch Zentrifugation (1 min bei 10000 xg) durch die Membran hindurchgeführt und der Elutionsschritt wird zum Zwecke einer vollständigen Elution noch einmal wiederholt. Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 1 dargestellt. Es werden jeweils die Mittelwerte der Doppelbestimmung angegeben.

**Tabelle 1: RNA-Ausbeute und Qualität nach photometrischer Messung**

| Transitionslösung | Lagerung | OD260/OD280 | RNA-Ausbeute/µg |
|---|---|---|---|
| N,N-Dimethylacetamid | 3d 2-8°C | 1,88 | 63,2 |
| | 1d RT | 1,88 | 61,9 |
| N,N-Diethylacetamid | 3d 2-8°C | 1,85 | 59,2 |
| | 1d RT | 1,85 | 59,1 |
| N,N-Diethylformamid | 3d 2-8°C | 1,79 | 47,8 |
| | 1d RT | 1,78 | 96,1 |
| | 3d RT | 1,80 | 55,6 |
| Dimethylthioformamid | 3d 2-8°C | 1,82 | 66,4 |
| | 1d RT | 1,79 | 47,1 |
| | 3d RT | 1,80 | 43,0 |

Die Ergebnisse zeigen, dass die erfindungsgemäßen Lösungen erfolgreich zur Stabilisierung von RNA in Transitionsexperimenten eingesetzt werden können

### Vergleichsbeispiel 2: Transition mit Mischungen mit N,N-Dimethylacetamid

Lebergewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit verschiedenen nicht-gekühlen Lösungen (s. Tabelle 2) versetzt und für 3 Tage bei 25°C gelagert. Im Anschluss an die Transition wird die RNA isoliert wie in Beispiel 1 beschrieben.

Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 2 dargestellt. Es werden jeweils die Mittelwerte der Doppelbestimmung angegeben.

**Tabelle 2: RNA-Ausbeute und Qualität nach photometrischer Messung**

| Transitionslösung | Lagerung | OD260/ OD280 | RNA-Ausbeute/ µg |
|---|---|---|---|
| 75% N,N-Dimethylacetamid + 25% DMSO | 3d 25°C | 1,71 | 21,9 |
| 75% N,N-Dimethylacetamid + 25% Aceton | 3d 25°C | 1,75 | 39,1 |
| 50% N,N-Dimethylacetamid + 50% Aceton | 3d 25°C | 1,74 | 36,2 |
| 25% N,N-Dimethylacetamid + 75% Aceton | 3d 25°C | 1,76 | 52,9 |
| 75% N,N-Dimethylacetamid + 25% Methanol | 3d 25°C | 1,73 | 44,5 |
| 50% N,N-Dimethylacetamid + 50% Methanol | 3d 25°C | 1,76 | 51,7 |
| 25% N,N-Dimethylacetamid + 75% Methanol | 3d 25°C | 1,80 | 54,1 |
| 75% N,N-Dimethylacetamid + 25% Diethylenglycol | 3d 25°C | 1,80 | 27,0 |
| 25% N,N-Dimethylacetamid + 75% Diethylenglycol | 3d 25°C | 1,77 | 20,8 |

Die Ergebnisse zeigen, dass die erfindungsgemäßen Reagenzien auch in Mischungen mit anderen Reagenzien, wie ein- und/oder mehrwertigen Alkoholen, DMSO oder Aldehyden erfolgreich eingesetzt werden können.

### Vergleichsbeispiel 3: Transition mit Mischungen aus N,N-Dimethylacetamid und Phenol

Lebergewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit verschiedenen nicht-gekühlen Mischungen aus N,N-Diemthylacetamid und Phenol (s. Tabelle 3) versetzt und für 3 Tage bei Raumtemperatur gelagert. Im Anschluss an die Transition wird die RNA isoliert wie in Beispiel 1 beschrieben. Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 3 dargestellt. Es werden jeweils die Mittelwerte der Doppelbestimmung angegeben.

**Tabelle 3: RNA-Ausbeute und Qualität nach photometrischer Messung**

| Transitionslösung | Lagerung | OD260/ OD280 | RNA-Ausbeute/ µg |
|---|---|---|---|
| N,N-Dimethylacetamid + 10% Phenol | 3d RT | 1,77 | 40,1 |
| N,N-Dimethylacetamid + 20% Phenol | 3d RT | 1,77 | 38,7 |
| N,N-Dimethylacetamid + 30% Phenol | 3d RT | 1,80 | 51,2 |
| N,N-Dimethylacetamid + 45% Phenol | 3d RT | 1,80 | 41,6 |

Die Ergebnisse zeigen, dass erfindungsgemäße Lösungen auch mit Phenol als Additiv erfolgreich zur Transition eingesetzt werden können.

### Vergleichsbeispiel 4: Verwendung von N,N-Dimethylacetamid als Additiv zur Verbesserung von Stabilisierungseigenschaften

Lebergewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit verschiedenen, nicht-gekühlten Lösungen versetzt und für 3 Tage bei 2-8°C im Kühlschrank oder für 1 Tag bei Raumtemperatur gelagert (siehe Tabelle 4). Im Anschluß an die Transition wird die RNA isoliert wie in Beispiel 1 beschrieben. Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 5 µl des Eluates eingesetzt.

**Tabelle 4**

| Nr | Transitionslösung | Lagerung |
|---|---|---|
| 1 | 100% Diethylenglycol | 3d 2-8°C |
| 2 | 95% Diethylenglycol + 5% N,N-Dimethylacetamid | 3d 2-8°C |
| 3 | 100% Triethylenglycol | 1d RT |
| 4 | 95% Triethylenglycol + 5% N,N-Dimethylacetamid | 1d RT |
| 5 | 100% 1,2,3-Propantriol | 3d 2-8°C |
| 6 | 95% 1,2,3-Propantriol + 5% N,N-Dimethylacetamid | 3d 2-8°C |
| 7 | 100% 1,5-Pentandiol | 1d RT |
| 8 | 100% 1,5-Pentandiol | 3d 2-8°C |
| 9 | 95% 1,5-Pentandiol + 5% N,N-Dimethylacetamid | 1d RT |
| 10 | 95% 1,5-Pentandiol + 5% N,N-Dimethylacetamid | 3d 2-8°C |

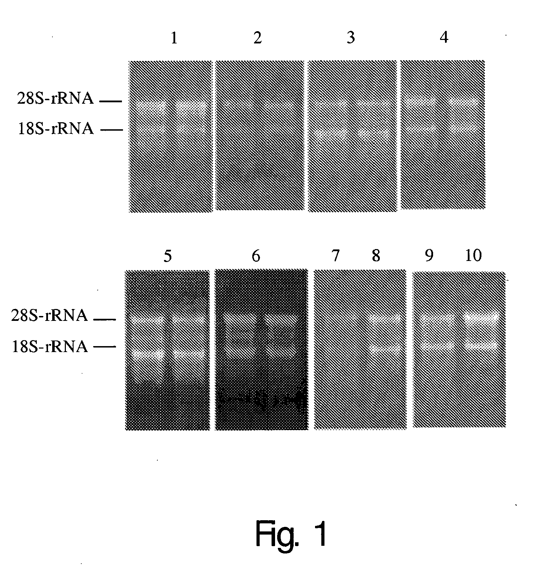

Die Ergebnisse sind in Fig. 1 wiedergegeben. Die Gele 2, 4, 6, 9 und 10 enthalten mit N,N-Dimethylacetamid und ggf. anderen Konservierungssubstanzen versetzte Proben, während die übrigen Gele unbehandelte bzw. nur mit anderen Konservierungssubstanzen behandelte Proben enthalten. Es ist erkennbar, dass hier im Vergleich zu den anderen Gelen (1, 3, 5, 7 und 8) der RNA-Gehalt größer und insbesondere die Qualität der RNA besser ist. RNA-Abbau ist in Gelanalysen erkennbar an der Abnahme der 28SrRNA im Vergleich zur 18SrRNA. Das native Verhältnis zwischen 28SrRNA und 18SrRNA beträgt etwa 2:1. Bei fortschreitendem Abbau verändert sich das Verhältnis dieser beiden rRNA-Banden zu 1:1, später zu 1:2 und im folgenden Verlauf zu einem vollständigen Verschwinden der 28SrRNA-Bande und später auch der 18SrRNA-Bande. Außerdem können bei RNA-Abbau auch weitere Banden im Gel sichtbar werden, bei denen es sich um rRNA-Abbau-Prödukte spezifischer Größe handelt. Zudem führt RNA-Abbau zum Auftreten eines niedermolekularen "Schmiers", bestehend aus degradierten RNA-Fragmenten, der insbesondere im Bereich unterhalb der 18SrRNA sichtbar wird, Die Gele 1, 3, 5, 7 und 8 zeigen die verschiedenen Merkmale von RNA-Abbau, während die korrespondierenden Gele 2, 4, 6, 9 und 10 (nach Zugabe von N,N-Dimethylacetamid zu den Konservierungssubstanzen), eine deutlich bessere RNA-Qualität aufweisen.

N,N-Dimethylacetamid kann also nicht nur allein, sondern auch als Additiv in geringer Menge zu anderen Konservierungssubstanzen zugegeben werden und dabei die Stabilisierungseigenschaften verbessern.

### Vergleichsbeispiel 5: Langzeitlagerung mit N,N-Dimethylacetamid

Nierengewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit verschiedenen nicht-gekühlen Lösungen (s. Tabelle 5) versetzt und für 3 Tage und 5 Tage bei 25°C gelagert. Als Kontrolle dient Gewebe, welches nach Entnahme in flüssigem Stickstoff eingefroren und bei -80°C gelagert wurde. Im Anschluss an die Transition wird die RNA isoliert wie in Beispiel 1 beschrieben. Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 5 dargestellt. Es werden jeweils die Mittelwerte der Doppelbestimmung angegeben.

**Tabelle 5: RNA-Ausbeute und Qualität nach photometrischer Messung**

| Nr | Transitionslösung | Lagerung | OD260/ OD280 | RNA-Ausbeute/ µg |
|---|---|---|---|---|
| 1 | Gefrorenes Gewebe | | 1,87 | 22,9 |
| 2 | N,N-Dimethylacetamid | 3d 25°C | 1,90 | 29,0 |
| 3 | | 5d25°C | 1,91 | 26,0 |
| 4 | 50% N,N-Dimethylacetamid + 50% Methanol | 3d 25°C | 1,93 | 27,9 |
| 5 | | 5d25°C | 1,97 | 28,9 |
| 6 | 25% N,N-Dimethylacetamid + 75% Methanol | 3d 25°C | 1,91 | 27,6 |
| 7 | | 5d25°C | 1,98 | 23,1 |

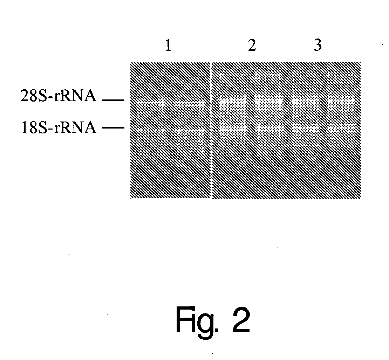

Zusätzlich wird exemplarisch die isolierte RNA der Proben 1 bis 3 auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 5 µl des Eluates eingesetzt. Die Ergebnisse sind in Fig. 2 wiedergegeben. Es ist deutlich erkennbar, dass die Zugabe von N,N-Dimethylacetamid eine Lagerung für längere Zeit bei höheren Temperaturen ermöglicht, ohne dass die Ausbeute, Qualität oder Integrität der RNA leidet.

### Vergleichsbeispiel 6: Transition mittels Mischungen mit N,N-Diethylacetamid

Lebergewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit verschiedenen nicht-gekühlen Mischungen aus N,N-Diemthylacetamid und Phenol (s. Tabelle 6) versetzt und für 1 Tag bei Raumtemperatur bwz. 3 Tage bei 2-8°C im Kühlschrank gelagert. Im Anschluss an die Transition wird die RNA isoliert wie in Beispiel 1 beschrieben. Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 6 dargestellt.

**Tabelle 6: RNA-Ausbeute und Qualität nach photometrischer Messung**

| Transitionslösung | Lagerung | OD260/ OD280 | RNA-Ausbeute/ µg |
|---|---|---|---|
| 50% N,N-Diethylacetamid + 50% Ethanol | 3d 2-8°C | 1,92 | 33,3 |
| 75% N,N-Diethylacetamid + 25% DMSO | 3d 2-8°C | 1,88 | 33,4 |
| 50% N,N-Diethylacetamid + 50% DMSO | 3d 2-8°C | 1,95 | 30,5 |
| | 1d RT | 1,86 | 39,0 |
| 25% N,N-Diethylacetamid + 75% DMSO | 3d 2-8°C | 1, 91 | 37,4 |
| | 1d RT | 1, 94 | 47,1 |
| 75% N,N-Diethylacetamid + 25% Aceton | 3d 2-8°C | 1,85 | 32,6 |
| 50% N,N-Diethylacetamid + 50% Aceton | 3d 2-8°C | 1,91 | 29,1 |
| 25% N,N-Diethylacetamid + 75% Aceton | 3d 2-8°C | 1, 84 | 31,9 |
| 75% N,N-Diethylacetamid + 25% Diethylenglycol | 3d 2-8°C | 1,85 | 31,4 |
| 50% N,N-Diethylacetamid + 50% Diethylenglycol | 3d 2-8°C | 1,87 | 44,2 |
| 25% N,N-Diethylacetamid + 75% Diethylenglycol | 3d 2-8°C | 1,92 | 42,2 |
| 75% N,N-Diethylacetamid + 25% Ethylenglycol | 3d 2-8°C | 1,84 | 41,3 |
| 50% N,N-Diethylacetamid + 50% 1,2-Propandiol | 3d 2-8°C | 1,86 | 40,5 |
| 90% N,N-Diethylacetamid + 10% Methanol | 3d 2-8°C | 1,84 | 41,7 |
| | 1d RT | 1,78 | 25,7 |
| 95% N,N-Diethylacetamid + 5% | 3d 2-8°C | 1,88 | 35,2 |
| N,N-Dimethylacetamid | 1d RT | 1,88 | 38,7 |
| 25% N,N-Diethylacetamid + 75% Glycerin | 3d 2-8°C | 1,89 | 44,0 |
| 50% N,N-Diethylacetamid + 50% Glycerin | 3d 2-8°C | 1,91 | 42,8 |
| 25% N,N-Diethylacetamid + 75% 1,3-Propandiol | 3d 2-8°C | 1,88 | 22,1 |
| | 1d RT | 1, 94 | 25,1 |
| 50% N,N-Diethylacetamid + 50% 1,3-Propandiol | 3d 2-8°C | 1,84 | 40,5 |
| 75% N,N-Diethylacetamid + 25% 2,3-propandiol | 3d 2-8°C | 1,91 | 39,9 |
| 25% N,N-Diethylacetamid + 75% einer 11,2M Dihydroxyaceton-Lösung (in Wasser) | 3d 2-8°C | 1,82 | 36,2 |
| 25% N,N-Diethylacetamid + 75% Triethylenglycol | 3d 2-8°C | 1, 86 | 45,5 |
| | 1d RT | 1,85 | 38,8 |
| 50% N,N-Diethylacetamid + 50% Triethylenglycol | 3d 2-8°C | 1,82 | 36,9 |
| 75% N,N-Diethylacetamid + 25% Triethylenglycol | 3d 2-8°C | 1, 84 | 45,9 |
| 25% N,N-Diethylacetamid + 75% 1,2,6-Hexantriol | 3d 2-8°C | 2,03 | 38,0 |
| 50% N,N-Diethylacetamid + 50% 1,2,6-Hexantriol | 3d 2-8°C | 1,93 | 39,4 |
| 25% N,N-Diethylacetamid + 75% 1,5-Pentandiol | 1d RT | 2,02 | 43,2 |
| | 3d 2-8°C | 1,91 | 49,5 |
| 50% N,N-Diethylacetamid + 50% 1,5-Pentandiol | 1d RT | 1,96 | 35,9 |
| | 3d 2-8°C | 1, 94 | 46,7 |
| 75% N,N-Diethylacetamid + 25% 1,5-Pentandiol | 1d RT | 1,91 | 41,3 |
| | 3d 2-8°C | 1,92 | 43,7 |
| 50% N,N-Diethylacetamid + 50% 2,4-Pentandiol | 3d 2-8°C | 1,97 | 32,2 |
| 75% N,N-Diethylacetamid + 25% 2,4-Pentandiol | 3d 2-8°C | 1,99 | 39,2 |
| 25% N,N-Diethylacetamid + 75% 1-Methoxy-2-Propanol | 3d 2-8°C | 2,05 | 38,3 |
| 50% N,N-Diethylacetamid + 50% 1-Methoxy-2-Propanol | 3d 2-8°C | 2,01 | 40,6 |
| 75% N,N-Diethylacetamid + 25% 1-Methoxy-2-Propanol | 3d 2-8°C | 2,05 | 48,1 |

Die Ergebnisse zeigen, dass Mischungen von N,N-Diethylacetamid mit verschiedenen anderen Konservierungssubstanzen erfolgreich RNA in Transitionsexperimenten stabilisieren.

### Vergleichsbeispiel 7: Langzeitlagerung mit N,N-Diethylacetamid

Nierengewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit verschiedenen nicht-gekühlen Lösungen (s. Tabelle 7) versetzt und für 3 und 5 Tage bei 25°C gelagert. Als Kontrolle dient Gewebe, welches nach Entnahme in flüssigem Stickstoff eingefroren und bei -80°C gelagert wurde. Im Anschluss an die Transition wird die RNA isoliert wie in Beispiel 1 beschrieben.

Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 7 dargestellt.

**Tabelle 7: RNA-Ausbeute und Qualität nach photometrischer Messung**

| Transitionslösung | Lagerung | OD260/ OD280 | RNA-Ausbeute/ µg |
|---|---|---|---|
| Gefrorenes Gewebe | | 1,97 | 23,1 |
| 25% Ethanol + 75% N,N-Diethylacetamid | 3d 25°C | 1,94 | 20,9 |
| | 5d25°C | 2,1 | 18,3 |
| 25% DMSO + 75% N,N-Diethylacetamid | 3d 25°C | 1,97 | 25,3 |
| | 5d25°C | 2,03 | 18,8 |
| 75% Aceton + 25% N,N-Diethylacetamid | 3d 25°C | 1,96 | 15,8 |
| | 5d25°C | 2,06 | 17,6 |
| 25% Diethylenglycol + 75% N,N-Diethylacetamid | 3d 25°C | 2,02 | 22,7 |
| | 5d25°C | 1, 99 | 20,5 |
| 50% N,N-Diethylacetamid + 50% 2,4-Pentandiol | 3d RT | 2,00 | 26,4 |
| 75% N,N-Diethylacetamid + 25% 2,4-Pentandiol | 3d RT | 1,89 | 27,3 |
| 25% N,N-Diethylacetamid + 75% 1-Methoxy-2-Propanol | 3d RT | 1, 99 | 21,1 |
| 50% N,N-Diethylacetamid + 50% 1-Methoxy-2-Propanol | 3d RT | 2,00 | 23,8 |
| 75% N,N-Diethylacetamid + 25% 1-Methoxy-2-Propanol | 3d RT | 1, 96 | 20,5 |

Die Ergebnisse zeigen, dass erfindungsgemäße Lösungen und Mischungen auch eine Lagerung für längere Zeit bei höheren Temperaturen ermöglichen.

### Beispiel 8: Stabilisierung von RNA in Zellkulturen

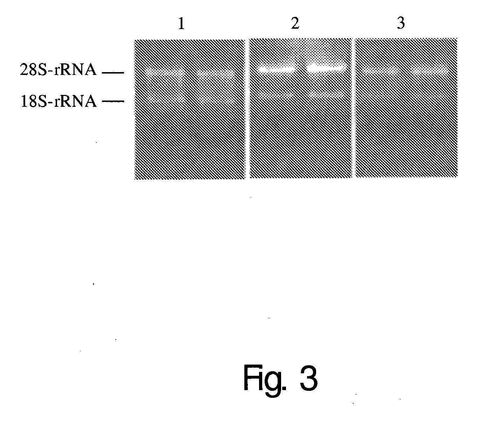

Jurkat-Zellkulturen werden bis zu einer Dichte von 5,4x10e5 Zellen je ml angezogen. Je 2 ml dieser Zellkultur werden für jede Probe des Experimentes abzentrifugiert und das Medium abgenommen. Die Zellpellets werden entweder in 500µl N,N-Diethylacetamid gelöst (Probe 2) oder in 500ul PBS gelöst und mit 5ml N,N.Diethylacetamid vermischt (Probe 3). Die mit N,N-Diethylacetamid in Kontakt gebrachten Zellen werden für 2 Tage bei Raumtemperatur gelagert. Als Kontrolle (Probe 1) dienen 2 ml der Zellkultur, die abzentrifugiert werden und nach Abnahme des Mediums direkt zur RNA-Isolierung ohne vorhergehende Lagerung verwendet werden. Zur RNA-Isolierung werden die Zellen durch Zentrifugation für 5 min bei 1500xg pelletiert und der Überstand verworfen. Die Pellets werden jeweils in 800µl des Puffers RLT der Firma QIAGEN durch Vortexen gelöst. Das Lysat wird mit 800µl 70% Ethanol vermischt und zweimal je 700µl des Gemisches werden auf 2 getrennte RNeasy-Mini Säulen des Herstellers QIAGEN aufgetragen und das Lysat durch Zentrifugation für 1 min bei 10000 x g durch die Membran geführt. Die weitere RNA-Aufreinigung erfolgt wie in Beispiel 1 beschrieben. Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 5 µl des Eluates eingesetzt. Die Ergebnisse sind in Fig. 3 wiedergegeben. Es zeigt sich dass N,N-Diethylacetamid auch RNA in frischen Zellkulturen, sowohl in Form von Pellets, als auch in Form von Suspensionen, erfolgreich stabilisiert.

### Vergleichsbeispiel 9: Transisitionsdauer

Lebergewebe der Ratte, welches nach Entnahme in flüssigem Stickstoff eingefroren und bei -80°C gelagert wurde, wird für diesen Versuch verwendet. Für jedes Transitionsexperiment werden 20 bis 50 mg Gewebe abgewogen und gefroren mit N,N-Dimemthylacetamid versetzt und für jeweils 2,5h, 6,5h und 22h bei 2-8°C im Kühlschrank und bei Raumtemperatur inkubiert (siehe Tabelle 8). Im Anschluss an die Transition wird die RNA isoliert wie in Beispiel 1 beschrieben.

Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 8 dargestellt.

**Tabelle 8: RNA-Ausbeute und Qualität nach photometrischer Messung**

| Nr | Lagerungstemperatur | Lagerungsdauer | OD260/ OD280 | RNA-Ausbeute/ µg |
|---|---|---|---|---|
| 1 | 2-8°C | 2,5h | 1,91 | 45,9 |
| 2 | | 6,5h | 1,97 | 43,0 |
| 3 | | 22h | 1,94 | 40,0 |
| 4 | Raumtempenatur | 2,5h | 1,93 | 50,5 |
| 5 | | 6,5h | 2,04 | 56,9 |
| 6 | | 22h | 1,99 | 44,4 |

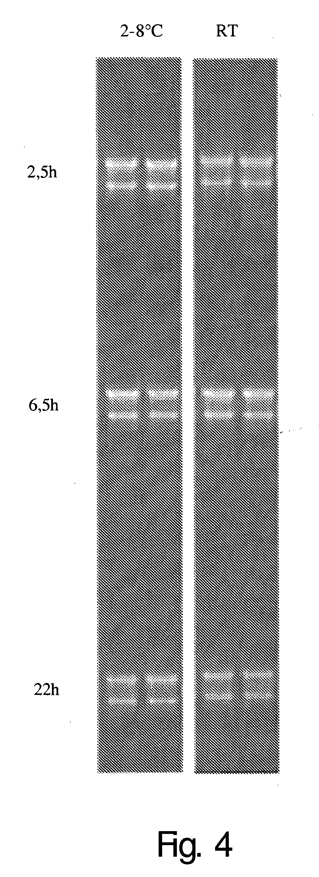

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 5 µl des Eluates eingesetzt. Die Ergebnisse sind in Fig. 4 wiedergegeben und zeigen, dass die Transition in N,N-Dimethylacetamid bei verschiedenen Temperaturen auch schon noch kurzer Zeit erfolgreich ist.

### Vergleichsbeispiel 10: Lagerung der Proben außerhalb der Transitionslösung

Lebergewebe der Ratte, welches nach Entnahme in flüssigem Stickstoff einfroren und bei -80°C gelagert, wird für diesen Versuch verwendet. Als Transitionslösung wird eine Lösung aus 50% N,N.Dieethylacetamid und 50% DMSO verwendet. Für das Transitionsexperiment wrid ca. 150 mg Gewebe abgewogen und gefroren mit der auf im Kühlschrank auf 2°C bis 8°C vorgekühlten Lösung o. g. Lösung versetzt. Die Probe wird über Nacht bei 2°-8°C im Kühlschrank gelagert.

Nach der Transition wird die Probe aus der Transitionslösung entnommen, zerteilt und die Teile (ca. 10-30mg) trocken bei Raumtemperatur für 30 min (1), 45 min (2), 60 min (3), 90 min (4), 2h 30 min (5) und 4h 30 min (6) gelagert. Anschließend wird die RNA wie unter Beispiel 1 beschrieben isoliert, wobei für diesen Versuch nur Einzelbestimmungen durchgeführt werden.

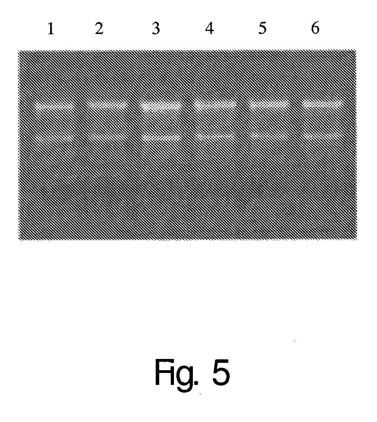

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 5 µl des Eluates eingesetzt. Die Ergebnisse sind in Fig. 5 wiedergegeben und zeigen, dass die Probe nach Transition auch über längere Zeit außerhalb der Transitionslösung aufbewahrt werden kann.

### Vergleichsbeispiel 11: Stabilisierung von RNA in frischen Gewebeproben

Frische Gewebe der Ratte werden direkt nach Entnahme für dieses Experiment verwendet. Für jedes Stabilisierungsexperiment werden ca. 20 bis 50 mg Gewebe mit unterschiedlichen Lösungen versetzt und bei verschiedenen Temperaturen gelagert (siehe Tabelle 9). Im Anschluss an die Lagerung wird die RNA aus Leber wie in Beispiel 1 beschrieben isoliert. Bei Niere und Dünndarm wird die RNA wie folgt isoliert: Zur RNA-Isolierung wird das Gewebe nach Lagerung aus den Lösungen entfernt und je 5 mg Niere bzw. je 10 mg Dünndarm 500 µl eines handelsüblichen Guanidinium-Isothiocyanat-Puffers, wie z. B. RLT-Puffer der Firma QIAGEN zugeben. Die Probe wird mit Hilfe einer Kugelmühle, wie z. B. TissueLyzer der Firma QIAGEN, über einen Zeitraum von 2 x 5 min bei 25 Hz mit einer 5 mm Stahlkugel homogenisiert, wobei der Guanidinium-Isothiocyanat-Puffer auf aus dem Stand der Technik bekannte Weise die Zellen lysiert und die freigesetzten Proteine denaturiert. Anschließend werden die Lysate bei 14000 rpm für 3 min zentrifugiert. Vom Überstand werden 500 µl, die 5 mg Gewebe repräsentieren, abgenommen. Zu diesen Proben wird 1 Volumen (500 µl) 70 %-iges Ethanol zugefügt und durch mehrmaliges Auf- und Abpipettieren oder durch Vortexen über einen Zeitraum von ca. 5 s gemischt. Das Lysat wird anschließend in eine handelsübliche Silicamembran enthaltene 96well-Platte, wie z. B. RNeasy96 Plate der Firma QIAGEN, aufgetragen und durch Zentrifugation (4 min bei 6000 rpm) durch die Membran hindurchgeführt. Die RNA bleibt an der Membran gebunden und wird anschließend mit einem ersten handelsüblichen Guanidinium-Isothiocyanat-haltigen Waschpuffer, beispielsweise mit dem Puffer RW1 der Firma QIAGEN, gewaschen. Anschließend wird zur enzymatsichen Entfernung etwaiger gebundener Gesamt-DNA DNAseI in einen geeigneten Puffer auf die Säule aufgeben und für 15 in bei Raumtemperatur zwecks Abbau der gebundenen DNA inkubiert. Im Anschluss wird erneut mit einem ersten handelsüblichen Guanidinium-Isothiocyanat-haltigen Waschpuffer, beispielsweise mit dem Puffer RW1 der Firma QIAGEN, und danach mit einem zweiten Tris-haltigen bzw. Tris- und alkoholhaltigen Waschpuffer, z. B. Puffer RPE der Firma QIAGEN, gewaschen. Dabei werden die Waschpuffer jeweils durch Zentrifugation (4 min bei 6000 rpm) durch die Membran hindurchgeführt. Die Waschung mit dem zweiten Tris-haltigen bzw. Tris- und alkoholhaltigen Waschpuffer wird wiederholt wobei gleichzeitig die Membran durch die Zentrifugation (10 min 6000 rpm) getrocknet wird. Zur Elution werden 50 µl RNase-freies Wasser auf die Membran pipettiert, um die gereinigte RNA von der Membran abzulösen. Nach einer Inkubation von 1 Minute bei einer Temperatur im Bereich von 10 - 30 °C wird das Eluat durch Zentrifugation (1 min bei 10000 xg) durch die Membran hindurchgeführt und der Elutionsschritt wird zum Zwecke einer vollständigen Elution noch einmal wiederholt. Die Menge an isolierter Gesamt-RNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 9 dargestellt.

**Tabelle 9: RNA-Ausbeute und Qualität nach photometrischer Messung**

| Nr | Transitionslösung | Gewebe | Lagerung | OD260/ OD280 | RNA-Ausbeute/ µg |
|---|---|---|---|---|---|
| 1 | Gefrorenes Gewebe | | | | |
| 2 | 50% N,N-Diethylacetamid + 50% DMSO | Niere | 1d 37°C | 2,06 | 11,2 |
| 3 | | Niere | 7d 2-8°C | 2,08 | 17,9 |
| 4 | | Dünndarm | 7d 25°C | 1,98 | 13,1 |
| 5 | 25% N,N-Diethylacetamid + 75% DMSO | Niere | 1d 37°C | 2,08 | 12,0 |
| 6 | | Niere | 7d 25°C | 2,11 | 14,1 |
| 7 | | Niere | 3d 2-8°C | 2,08 | 11,0 |
| 8 | | Niere | 7d 2-8°C | 2,09 | 19,4 |
| 9 | | Dünndarm | 7d 25°C | 2,12 | 20,3 |
| 10 | | Dünndarm | 7d 2-8°C | 2,08 | 29,6 |
| 11 | 5% N,N-Dimethylacetamid + 95% Ethanol + Essigsäure (40µl auf 20ml gesamt-Volumen) | Leber | 3d 25°C | 1,926 | 45,7 |
| 12 | 75% N,N-Diethylacetamid + 25% Diethylenglycol | Leber | 3d 25°C | 1,99 | 59,3 |
| 13 | 25% N,N-Diethylacetamid + 75% 2,4-Pentandiol | Leber | 3d 25°C | 1,89 | 60,3 |
| 14 | 25% N,N-Diethylacetamid + 75% 1,2,3-Propantriol | Leber | 3d 25°C | 2,1 | 64,4 |

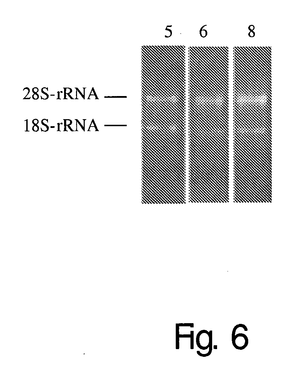

Zusätzlich wird exemplarisch die isolierte RNA einiger Proben auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 5 µl des Eluates eingesetzt. Die Ergebnisse sind in Fig. 6 wiedergegeben und zeigen, dass die erfindungsgemäßen Lösungen auch RNA in frischem Gewebe erfolgreich stabilisieren können, wobei die Lagerung über längere Zeiträume und bei hohen Temperaturen erfolgen kann.

### Vergleichsbeispiel 12: Stabilisierung von DNA in frischen Gewebeproben

Nierengewebe der Ratte wurde unverzüglich nach Organentnahme mit Mischungen aus N,N-Diethylacetamid und DMSO versetzt und bei verschiedenen Temperaturen gelagert (siehe Tabelle 10). Im Anschluss an die Lagerung wird die DNA aus den gelagerten Proben isoliert. Zur DNA-Isolierung wird das Gewebe nach Lagerung aus den Lösungen entfernt und je 10 mg Gewebe in 180 µl des Puffers ALT des Herstellers QIAGEN zugeben. Die Probe wird mit Hilfe einer Kugelmühle, wie z. B. TissueLyzer der Firma QIAGEN, über einen Zeitraum von 30 sek bei 25 Hz mit einer 5 mm Stahlkugel homogenisiert und anschließend für 15 Sekunden bei 14000xg zentrifugiert. Nach Zugabe von 120µl der Protease K-Lösung (Hersteller QIAGEN) werden die Lysate für 2 Stunden bei 55°C unter schütteln inkubiert. Nach der Inkubation werden 4µl RNAse A (100mg/ml) zugeben, gemischt und die Mischung für 2 min bei Raumtemperatur inkubiert. Nach der Inkubation wird 300µl eines handelsüblichen Guanidinium-Hydrochloridhaltigen Lysepuffers, wie der Puffer AL des Herstellers QIAGEN, zugegeben und die Proben mittels Vortexen durchmischt. Es erfolgt eine Inkubation bei 70°C für 10 min. Nach Mischung mit 300µl 100% Ethanol werden die Proben auf eine Silicamembran enthaltende 96well-Platte (DNeasy 96 plate der Firma QIAGEN) aufgetragen und das Lysat mittels Zentrifugaion für 10 min bei 6000 rpm durch die Membran geführt. Die DNA bleibt an der Membran gebunden und wird zunächst mit einem ersten handelsüblichen Guanidinium-Hydrochloridhaltigen Waschpuffer, beispielsweise mit dem Puffer AW1 der Firma QIAGEN, und danach mit einem zweiten alkoholhaltigen Waschpuffer, z. B. Puffer AW2 der Firma QIAGEN, gewaschen. Dabei werden die Waschpuffer jeweils durch Zentrifugation (5 min bei 6000rpm) durch die Membran hindurchgeführt Im Anschluss wird die Platte für 10 min bei 70°C inkubiert. Die Elution der DNA erfolgt durch Auftrag von 200µl des auf 70°C vorgewärmten Elutionspuffers AE (QIAGEN). Nach einminütiger Inkubation wird der Elutionspuffer durch Zentrifugation durch die Membran hindurchgeführt (5 min bei 6000 rpm) und die Elution wiederholt. Die Menge an isolierter Gesamt-DNA wird nach Verdünnung in Wasser durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt. Die Ergebnisse der Isolierungen sind in Tabelle 10 dargestellt.

**Tabelle 10: DNA-Ausbeute und Qualität nach photometrischer Messung**

| Transitionslösung | Lagerung | OD260/ OD280 | RNA-Ausbeute/ µg |
|---|---|---|---|
| 50% DMSO + 50% N,N-Diethylacetamid | 1d 37°C | 1,96 | 17,9 |
| | 3d 25°C | 1,93 | 12,8 |
| | 3d 2-8°C | 1,97 | 15,0 |
| | 7d 25°C | 2,01 | 19,84 |
| | 7d 2-8°C | 1,99 | 24,9 |
| 75% DMSO + 25% N,N-Diethylacetamid | 1d 37°C | 1,97 | 17,5 |
| | 3d 25°C | 1,96 | 13,3 |
| | 3d 2-8°C | 1,97 | 13,21 |
| | 7d 25°C | 1,98 | 17, 61 |
| | 7d 2-8°C | 2,03 | 26,9 |

Die Ergebnisse zeigen, dass die erfindungsgemäßen Lösungen auch DNA in frischem Gewebe erfolgreich stabilisieren können, wobei die Lagerung über längere Zeiträume und bei hohen Temperaturen erfolgen kann.

### Vergleichsbeispiel 13: Histologische Analyse von stabilisierten Geweben

Leber und Nierengewebe der Ratte wurde unverzüglich nach Organentnahme mit je 1 ml 25% N,N-Diethylacetamid + 75% DMSO versetzt und für 1 Tag bei 25°C im Inkubator gelagert. Nach der Lagerung: werden die Gewebestücke aus den Lösungen entnommen, in Plastikkassetten überführt und nach üblichen Protokollen in einer aufsteigenden Ethanolreihe, sowie in Xylol inkubiert und in Paraffin eingebettet. Mit Hilfe eines Microtoms werden Schnitten aus den in Paraffin eingebetteten Gewebe erstellt und diese auf dem Objektträger nach üblichen Methoden einer Hämatoxylin-Eosin-Färbung unterzogen. Die gefärbten Gewebeschnitte werden lichtmikroskopisch untersucht, wobei erkennbar wird, dass die Lösung den Erhalt der Morphologie der Gewebe ermöglicht.

### Vergleichsbeispiel 14: Stabilisierung von Proteinen in frischen Gewebeproben

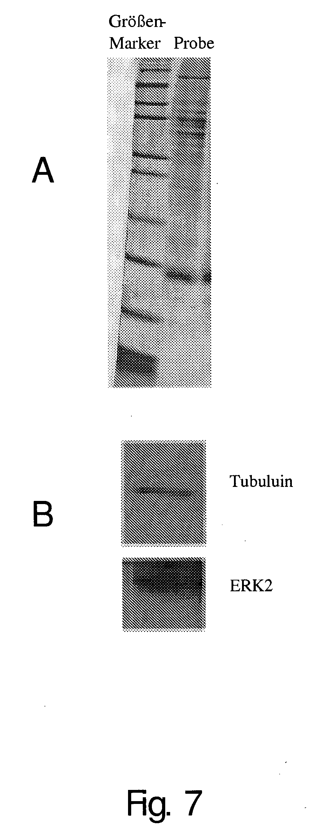

Lebergewebe der Ratte wurde unverzüglich nach Organentnahme mit 1 ml 25% N,N-Diethylacetamid + 75% DMSO versetzt und für 3 Tage bei Raumtemperatur gelagert. Im Anschluss an die Lagerung wird ein Proteinextrakt aus der gelagerten Probe hergestellt. Zur Herstellung des Proteinextraktes wird das Gewebe nach Lagerung aus den Lösungen entfernt und je 10 mg Gewebe 400µl eines üblichen Extraktionspuffers, hier in einer Zusammensetzung von 8M Harnstoff, 100mM Natriumdihydrogenphosphat und 10mM Tris, pH 8,0, zugeben und die Probe mit Hilfe einer Kugelmühle, z. B. dem TissueLyzer der Firma QIAGEN, homogenisiert. Das so entstandene Lysat wird für 15 sek. bei möglichst hoher Drehzahl (z. B. ca. 20000xg) zentrifugiert um ungelöste Bestandteile zu pelletieren. Der proteinhaltige Überstand wird abgenommen und die Proteinkonzentration mittels eines Bradford-testes bestimmt. 1,5µg Protein wird auf einem SDS-Polyacrylamidgel nach üblichem Verfahren aufgetrennt und zum einen das Gel mit Coomassie nach üblichen Verfahren angefärbt und zum anderen ein zweites Gel mittels einer Semidry-Blotting-Apparatur nach Angaben des Herstellers auf eine Nitrozellulosemembran geblottet. Die Membran wird nach dem Stand der Technik mit Milchpulver abgesättigt und mit einem ERK2-spezifischen Antikörper sowie einem tubulin-spezifischen Antikörper nach Angaben des Herstellers hybridisiert, und eine Immunodetektion durchgeführt. Die Ergebnisse sind in Fig. 7 dargestellt und zeigen, dass die Proteine durch die erfindungsgemäße Zusammensetzung bei Raumtemperatur in Geweben stabilisiert werden.

## Patentansprüche

1. Ein Verfahren zur Stabilisierung von Zellen und Biomolekülen in einer biologischen Probe, wobei die Stabilisierung ohne die Zerstörung der biologischen Probe erfolgt, aufweisend die folgenden Schritte:
a) Bereitstellen einer biologischen Probe enthaltend Zellen, und
b) in Kontakt bringen der biologischen Probe mit einer Zusammensetzung, aufweisend eine Substanz gemäß der folgenden Strukturformel: 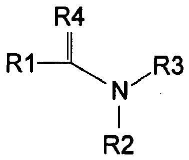 worin R1 ein Wasserstoffrest oder ein Methylrest ist, R2 und R3 gleiche oder unterschiedliche Kohlenwasserstoff-Reste mit einer Kohlenstoff-Kettenlänge von 1 - 20 sind, sowie R4 ein Sauerstoff-, ein Schwefel- oder ein Selenrest ist,
wobei es sich bei der biologischen Probe enthaltend Zellen um ein Material ausgewählt aus Körperflüssigkeiten, Blut, Leukozytenfraktionen, Crusta Phlogistica, Sputum, Speichel, Urin, Sperma, Faeces, Abstriche, Suspensionen, flüssige Proben, Plasma, Serum und Zellpellets handelt und wobei die Stabilisierung die Isolierung von Biomolekülen aus der stabilisierten Probe erlaubt.

2. Verfahren gemäß Anspruch 1, wobei es sich bei der biologischen Probe um eine gefrorene biologische Probe handelt.

3. Verfahren gemäß Anspruch 1, wobei es sich bei der biologischen Probe um eine nicht-gefrorene biologische Probe handelt.

4. Verfahren gemäß einem der vorhergehenden Ansprüche, wobei es sich bei der biologischen Probe enthaltend Zellen um ein Material ausgewählt aus Körperflüssigkeiten, Blut, Urin und Suspensionen handelt.

5. Verfahren gemäß einem der vorhergehenden Ansprüche, wobei es sich bei der Substanz um eine Substanz ausgewählt aus der Gruppe N,N-Dimethylacetamid, N,N-Diethylacetamid, N,N-Dimethylformamid, N,N-Diethylformamid, N,N-Dimethylthioforamid und N,N-Dimethylthioforamid handelt.

6. Verfahren gemäß einem der vorhergehenden Ansprüche, **dadurch gekennzeichnet, dass** die Probe außerdem mit einem oder mehreren Stoffen in Kontakt gebracht wird, ausgewählt aus der Gruppe enthaltend ein- und/oder mehrwertige Alkohole, Lösemittel, Puffersubstanzen Chelatoren, und Mittel zur Verbesserung der Sichtbarkeit des Zellkern.

7. Verfahren gemäß einem der vorhergehenden Ansprüche, aufweisend zusätzlich zu den Schritten a) und b) den folgenden Schritt:
c) Lagerung der mit der Zusammensetzung in Kontakt gebrachten biologischen Probe bei Raumtemperatur

8. Verfahren nach Anspruch 7, **dadurch gekennzeichnet, dass** es bei der Lagerung nicht zu einem Abbau von Nukleinsäuren in der biologischen Probe kommt.

9. Verfahren nach Anspruch 7 oder 8, **dadurch gekennzeichnet, dass** die Lagerung über einen Zeitraum erfolgt, der ausgewählt ist aus
i) mindestens einem Tag;
ii) mindestens zwei Tagen und
iii) mindestens drei Tagen.

10. Verfahren nach einem oder mehreren der Ansprüche 1 bis 9, **dadurch gekennzeichnet, dass** die biologische Probe ausgewählt ist aus Blut, Plasma und Serum.

11. Verfahren zur Analyse einer biologischen Probe, aufweisend die Schritte a) und b) sowie gegebenenfalls Schritt c) aus den Ansprüchen 1 - 10 , aufweisend zusätzlich die folgenden Schritte:
d) histologisch-morphologische Analyse der mit der Zusammensetzung in Kontakt gebrachten biologischen Probe; und/oder
e) Analyse von Biomolekülen in der oder aus der mit der Zusammensetzung in Kontakt gebrachten biologischen Probe.

12. Verfahren nach Anspruch 11, **dadurch gekennzeichnet, dass** in der stabilisierten Probe der Abbau, die Modifikation oder die Änderung der Aktivität von Biomolekülen gehemmt ist.

13. Verfahren nach Anspruch 11 oder 12, **dadurch gekennzeichnet, dass** die Analyse die Isolierung der Biomoleküle aus der mit der Zusammensetzung in Kontakt gebrachten Probe umfasst.

14. Verfahren nach einem oder mehreren der Ansprüche 11 bis 13, **dadurch gekennzeichnet, dass** es sich bei den Biomolekülen um Nukleinsäuren handelt.

15. Verwendung einer Zusammensetzung gemäß Anspruch 1 oder eines Kits of Parts aufweisend die Zusammensetzung gemäß Anspruch 1 in einem Verfahren nach einem der Ansprüche 1 - 14.

16. Verwendung einer Zusammensetzung gemäß Anspruch 1 zur Stabilisierung von Zellen in einer biologischen Probe, wobei die Stabilisierung ohne die Zerstörung der biologischen Probe erfolgt und wobei es sich bei der biologischen Probe enthaltend Zellen um ein Material ausgewählt aus Körperflüssigkeiten, Blut, Leukozytenfraktionen, Crusta Phlogistica, Sputum, Speichel, Urin, Sperma, Faeces, Abstriche, Suspensionen, flüssige Proben, Plasma, Serum und Zellpellets handelt und in der stabilisierten Probe der Abbau, die Modifikation oder die Änderung der Aktivität von Biomolekülen gehemmt ist und wobei die Stabilisierung die Isolierung von Biomolekülen aus der stabilisierten Probe erlaubt.

17. Verwendung nach Anspruch 16, wobei es sich bei der biologischen Probe enthaltend Zellen um ein Material ausgewählt aus Körperflüssigkeiten, Blut, Urin und Suspensionen handelt.

## Claims

1. Method for stabilizing cells and biomolecules in a biological sample, wherein the stabilization proceeds without the destruction of the biological sample, comprising the following steps:
a) providing a biological sample containing cells, and
b) contacting the biological sample with a composition comprising a substance according to the following structural formula: 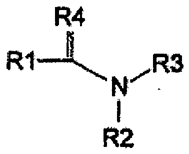 where R1 is a hydrogen residue or a methyl residue, R2 and R3 are identical or different hydrocarbon residues with a carbon chain length of 1-20, and R4 is an oxygen, a sulphur or a selenium residue,
wherein the biological sample containing cells is a material selected from body fluids, blood, leukocyte fractions, crusta phlogistica, sputum, saliva, urine, sperm, faeces, swabs, suspensions, liquid samples, plasma, serum and cell pellets, and wherein the stabilization permits the isolation of biomolecules from the stabilized sample.

2. Method according to Claim 1, wherein the biological sample is a frozen biological sample.

3. Method according to Claim 1, wherein the biological sample is a non-frozen biological sample.

4. Method according to one of the preceding claims, wherein the biological sample containing cells is a material selected from body fluids, blood, urine and suspensions.

5. Method according to one of the preceding claims, wherein the substance is a substance selected from the group N,N-dimethylacetamide, N,N-diethyl¬acetamide, N,N-dimethylformamide, N,N-diethylformamide, N,N dimethylthioformamide and N,N-dimethylthioformamide.

6. Method according to one of the preceding claims, **characterized in that** the sample is additionally contacted with one or more materials selected from the group containing monohydric and/or polyhydric alcohols, solvents, buffer substances, chelators and means for improving the visibility of the cell nucleus.

7. Method according to one of the preceding claims, comprising, in addition to steps a) and b), the following step:
c) storage of the biological sample that was contacted with the composition at room temperature.

8. Method according to Claim 7, **characterized in that**, during the storage, degradation of nucleic acids in the biological sample does not occur.

9. Method according to Claim 7 or 8, **characterized in that** the storage proceeds over a period which is selected from
i) at least one day;
ii) at least two days and
iii) at least three days.

10. Method according to one or more of Claims 1 to 9, **characterized in that** the biological sample is selected from blood, plasma and serum.

11. Method for analysis of a biological sample, comprising the steps a) and b), and optionally step c) from Claims 1-10, comprising additionally the following steps:
d) histological-morphological analysis of the biological sample which has brought into contact with the composition; and/or
e) analysis of biomolecules in, or from the, biological sample which was brought into contact with the composition.

12. Method according to Claim 11, **characterized in that** in the stabilized sample the degradation, modification or change in activity of biomolecules is inhibited.

13. Method according to Claim 11 or 12, **characterized in that** the analysis comprises the isolation of the biomolecules from the sample which was brought into contact with the composition.

14. Method according to one or more of Claims 11 to 13, **characterized in that** the biomolecules are nucleic acids.

15. Use of a composition according to Claim 1, or of a kit of parts comprising the composition according to Claim 1 in a method according to any one of Claims 1 14.

16. Use of a composition according to Claim 1 for stabilizing cells in a biological sample, wherein the stabilization proceeds without the destruction of the biological sample, and wherein the biological sample containing cells is a material selected from body fluids, blood, leukocyte fractions, crusta phlogistica, sputum, saliva, urine, sperm, faeces, swabs, suspensions, liquid samples, plasma, serum and cell pellets, and in the stabilized sample the degradation, the modification or the change in activity of biomolecules is inhibited, and wherein the stabilization permits the isolation of biomolecules from the stabilized sample.

17. Use according to Claim 16, wherein the biological sample containing cells is a material selected from body fluids, blood, urine and suspensions.

## Revendications

1. Procédé de stabilisation de cellules et de biomolécules dans un échantillon biologique, dans lequel la stabilisation se fait sans destruction de l'échantillon biologique, présentant les étapes suivantes :
a) mise à disposition d'un échantillon biologique contenant des cellules, et
b) mise en contact de l'échantillon biologique avec une composition présentant u ne substance répondant à la formule structurelle suivante : 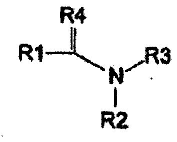 dans laquelle R1 est un groupe hydrogène ou un groupe méthyle, R2 et R3 sont des groupes hydrocarbure identiques ou différents présentant une longueur de chaîne hydrocarbonée comprise entre 1 et 20, et R4 est un résidu oxygène, soufre ou sélénium,
où il s'agit concernant l'échantillon biologique contenant des cellules d'un matériau sélectionné parmi les fluides corporels, le sang, les fractions leucocytaires, la croûte phlogistique, l'expectoration, la salive, l'urine, le sperme, les fèces, les frottis, les suspensions, les échantillons liquides, le plasma, le sérum, les culots cellulaires, et où la stabilisation permet l'isolement de biomolécules depuis l'échantillon stabilisé.

2. Procédé selon la revendication 1, dans lequel il s'agit concernant l'échantillon biologique d'un échantillon biologique congelé.

3. Procédé selon la revendication 1, dans lequel il s'agit concernant l'échantillon biologique d'un échantillon biologique non congelé.

4. Procédé selon l'une des revendications précédentes, dans lequel il s'agit concernant l'échantillon biologique contenant des cellules d'un matériau sélectionné parmi les fluides corporels, le sang, l'urine et les suspensions.

5. Procédé selon l'une des revendications précédentes, dans lequel il s'agit concernant la substance d'une substance sélectionnée dans le groupe composé du N,N-diméthylacétamide, du N,N-diéthylacétamide, du N,N-diméthylformamide, du N,N-diéthylformamide, du N,N-diméthylthioformamide, et du N,N-diéthylthioformamide.

6. Procédé selon l'une des revendications précédentes, **caractérisé en ce que** l'échantillon est en outre mis en contact avec une ou plusieurs substances sélectionnées dans le groupe contenant des alcools mono- ou polyvalents, des solvants, des substances tampon, des agents chélatants, et des agents permettant d'améliorer la visibilité du noyau cellulaire.

7. Procédé selon l'une des revendications précédentes, présentant en plus des étapes a) et b) les étapes suivantes :
c) stockage de l'échantillon biologique mis en contact avec la composition à la température ambiante.

8. Procédé selon la revendication 7, **caractérisé en ce que** le stockage ne provoque pas de décomposition d'acides nucléiques dans l'échantillon biologique.

9. Procédé selon la revendication 7 ou 8, **caractérisé en ce que** le stockage se fait sur une durée sélectionnée parmi
i) au moins un jour ;
ii) au moins deux jours; et
iii) au moins trois jours.

10. Procédé selon une ou plusieurs des revendications 1 à 9, **caractérisé en ce que** l'échantillon biologique est sélectionné parmi le sang, le plasma et le sérum.

11. Procédé d'analyse d'un échantillon biologique, comprenant les étapes a) et b) ainsi qu'éventuellement l'étape c) des revendications 1 à 10, et présentant en outre les étapes suivantes :
d) analyse histomorphologique de l'échantillon biologique mis en contact avec la composition, et/ou
e) analyse des biomolécules dans ou issues de l'échantillon biologique mis en contact avec la composition.

12. Procédé selon la revendication 11, **caractérisé en ce que** dans l'échantillon stabilisé, la dégradation, la modification ou le changement de l'activité des biomolécules est empêché.

13. Procédé selon la revendication 11 ou 12, **caractérisé en ce que** l'analyse comprend l'isolement des biomolécules depuis l'échantillon mis en contact avec la composition.

14. Procédé selon une ou plusieurs des revendications 11 à 13, **caractérisé en ce qu'**il s'agit concernant les biomolécules d'acides nucléiques.

15. Utilisation d'une composition selon la revendication 1 ou d'une trousse de parties présentant la composition selon la revendication 1 dans un procédé selon l'une des revendications 1 à 14.

16. Utilisation d'une composition selon la revendication 1 pour la stabilisation de cellules dans un échantillon biologique, où la stabilisation se fait sans la destruction de l'échantillon biologique et où il s'agit concernant l'échantillon biologique contenant des cellules d'un matériau sélectionné parmi les fluides corporels, le sang, les fractions leucocytaires, la croûte phlogistique, l'expectoration, la salive, l'urine, le sperme, les fèces, les frottis, les suspensions, les échantillons liquides, le plasma, le sérum ou les culots cellulaires, et où dans l'échantillon stabilisé, la dégradation, la modification ou le changement de l'activité des biomolécules est empêché, et où la stabilisation permet l'isolement des biomolécules depuis l'échantillon stabilisé.

17. Utilisation selon la revendication 16, dans lequel il s'agit concernant l'échantillon biologique contenant des cellules d'un matériau sélectionné parmi les fluides corporels, le sang, l'urine et les suspensions.
